# EUROPÄISCHE PATENTSCHRIFT

(11) **EP 0 958 276 B1**
(45) Veröffentlichungstag und Bekanntmachung des Hinweises auf die Patenterteilung: **13.03.2002**
(21) Anmeldenummer: 97953895.6
(22) Anmeldetag: 19.12.1997
(51) Int. Cl.: C07C 251/60, C07C 251/86, A01N 33/24, A01N 33/26

(54) **SUBSTITUIERTE 2-BENZOYL-CYCLOHEXAN-1,3-DIONE MIT HERBIZIDER WIRKUNG**
SUBSTITUTED 2-BENZOYL-CYCLOHEXAN-1,3-DIONES WITH HERBICIDAL EFFECT
2-BENZOYL-CYCLOHEXANE-1,3-DIONES SUBSTITUEES A ACTION HERBICIDE

(30) Priorität: 03.01.1997 DE 19700019
(43) Veröffentlichungstag der Anmeldung: 24.11.1999
(73) Patentinhaber: BASF AKTIENGESELLSCHAFT, 67056 Ludwigshafen (DE)
(72) Erfinder: HILL, Regina, Luise, 67376 Harthausen (DE); KARDORFF, Uwe, D-68159 Mannheim (DE); RACK, Michael, D-69123 Heidelberg (DE); BAUMANN, Ernst, D-67373 Dudenhofen (DE); VON DEYN, Wolfgang, D-67435 Neustadt (DE); ENGEL, Stefan, D-65510 Idstein (DE); MAYER, Guido, D-67433 Neustadt (DE); OTTEN, Martina, D-67069 Ludwigshafen (DE); RHEINHEIMER, Joachim, D-67063 Ludwigshafen (DE); WITSCHEL, Matthias, D-67061 Ludwigshafen (DE); MISSLITZ, Ulf, D-67433 Neustadt (DE); WALTER, Helmut, D-67283 Obrigheim (DE); WESTPHALEN, Karl-Otto, D-67346 Speyer (DE)
(86) Internationale Anmeldenummer: EP9707214
(87) Internationale Veröffentlichungsnummer: WO9829384

(56) Entgegenhaltungen:
- EP-A- 0 137 963
- WO-A-96/26200
- WO-A-97/46530
- DE-A- 2 326 462
- DATABASE CROSSFIRE beilstein RN = , XP002063835 & ,

## Beschreibung

Die vorliegende Erfindung betrifft substituierte 2-Benzoyl-cyclohexan-1,3-dione der Formel I 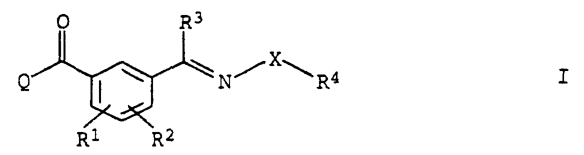 in der die Variablen folgende Bedeutung haben:
- R¹, R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵, -OCOR⁶, -OSO₂R⁶, -SH, -S(O)ₙR⁷, -SO₂OR⁵, -SO₂NR⁵R⁸, -NR⁸SO₂R⁶ oder -NR⁸COR⁶;
- R³: Wasserstoff, Cyano, C₁-C₆-Alkyl, C₁-C₆-Halogenalkyl. -OR⁷, -SR⁷ oder -NR⁷R¹⁰;
- R⁴: Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
- X: Sauerstoff oder NR⁸:
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁶: C₁-C₆-Alkyl oder C₁-C₆-Halogenalkyl;
- R⁷: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁸: Wasserstoff oder C₁-C₆-Alkyl;
- R⁹: C₁-C₆-Alkyl, C₃-C₆-Alkenyl, C₃-C₆-Alkinyl, Phenyl oder Benzyl;
- R¹⁰: C₁-C₆-Alkyl, C₁-C₆-Halogenalkyl, C₃-C₆-Alkenyl oder C₃-C₆-Alkinyl;
- Q: ein gegebenenfalls substituierter in 2-Stellung verknüpfter Cyclohexan-1,3-dionring;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

2-Benzoyl-cyclohexan-1,3-dione sind bekannt, beispielsweise aus EP-A 90262, EP-A 135 191, EP-A 186 118, EP-A 186 119, EP-A 186 120, EP-A 319 075, WO 90/05712 und JO 31 20 202. Diese Schriften lehren 2-Benzoyl-cyclohexan-1,3-dione, die in 3-Position unsubstituiert sind oder kleine Reste wie Halogen, Alkoxy, NO₂ etc. tragen.

Weitere 2-Benzoylcyclohexan-1,3-dione sind aus EP-A 278 742, EP-A 298 680, EP-A 320 864 und WO 96/14285 bekannt.

In WO 96/26 200 werden 2-Benzoyl-cyclohexan-1,3-dione offenbart, die in 3-Position des Phenylrestes eine heterocyclische Gruppe tragen.

Des weiteren lehrt JO 30 52 862 Cyclohexan-1,3-dione, die in 2-Position mit einem ggf. substituierten Pyridylrest verknüpft sind.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 2-Benzoyl-cyclohexan-1,3-dione der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon umfaßt.

Die Verbindungen der Formel I enthalten eine Kohlenstoff-Stickstoff-Doppelbindung und liegen daher als E-Isomere oder Z-Isomere oder als E/Z-Isomerengemische vor. Weiterhin können die Verbindungen der Formel I weitere Kohlenstoff-Kohlenstoff- bzw. Kohlenstoff-Stickstoff-Doppelbindungen enthalten. Gegenstand der Erfindung sind sowohl die reinen geometrischen Isomere als auch Gemische hiervon.

Die Verbindungen der Formel I können ebenso je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschstenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Hervorzuheben sind die erfindungsgemäßen Verbindungen der Formel I, wobei die Variable Q einen in 2-Stellung verknüpften Cyclohexan-1,3-dionring der Formel II darstellt, 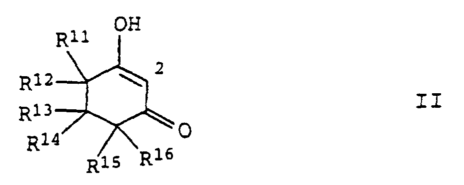 wobei II auch stellvertretend für die tautomeren Formeln II' und II'' steht, 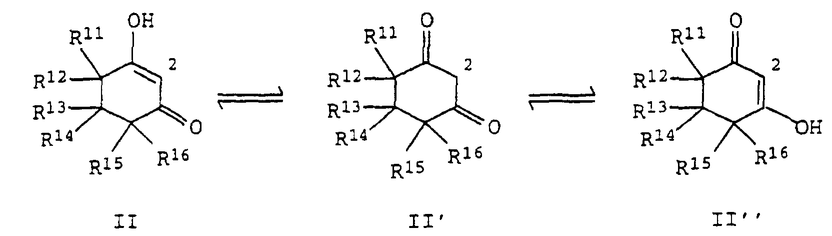 wobei
- R¹¹, R¹², R¹⁴ und R¹⁶: für Wasserstoff oder C₁-C₄-Alkyl stehen;
- R¹³: für Wasserstoff, C₁-C₄-Alkyl oder C₃-C₄-Cycloalkyl steht, wobei die beiden letztgenannten Gruppen einen bis drei der folgenden Substituenten tragen können. Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch ein bis drei C₁-C₄-Alkylreste substituiert sein können;
- R¹⁵: für Wasserstoff, C₁-C₄-Alkyl oder C₁-C₆-Alkoxycarbonyl steht;
oder
- R¹³ und R¹⁶: gemeinsam eine π-Bindung oder einen drei- bis sechsgliedrigen carbocyclischen Ring bilden;
oder
die CR¹³R¹⁴-Einheit durch C=O ersetzt sein kann.

Ebenso hervorzuheben sind die erfindungsgemäßen Verbindungen der Formel I, wobei
- R⁴: Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits partiell oder vollständig halogeniert sein können und/ oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
bedeuten.

Die für die Substituenten R¹-R¹⁶ oder als Reste an Phenyl-, Hetaryl- und Heterocylylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylthio, Alkylsulfonyl-, Alkylcarbonyl-, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, Propyl, 1-Methylethyl, Butyl, 1-Methylpropyl, 2-Methylpropyl und 1,1-Dimethylethyl;
- C₁-C₄-Alkyl, sowie die Alkylteile von C₁-C₄-Alkylcarbonyl: C₂-C₄-Alkyl, wie voranstehend genannt sowie Methyl;
- C₂-C₆-Alkyl, sowie die Alkylteile von C₁-C₆-Alkoxy-C₂-C₆-alkyl: C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₆-Alkyl, sowie die Alkylteile von C₁-C₆-Alkoxy-C₁-C₆-alkyl: C₂-C₆-Alkyl, wie voranstehend genannt, sowie Methyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylthio: Methylthio, Ethylthio, Propylthio, 1-Methylethylthio, Butylthio, 1-Methylpropylthio, 2-Methylpropylthio und 1,1-Dimethylethylthio;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₄-Alkyliminooxy: Methyliminooxy, Ethyliminooxy, 1-Propyliminooxy, 2-Propyliminooxy, 1-Butyliminooxy und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-l-yl, 2-Methyl-pent-3-en-1-yl, 3-Methyl-pent-3-en-1-yl, 4-Methyl-pent-3-en-1-yl, l-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heteroclylreste in Heterocyclyloxy:
   drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, Oxetan-3-yl, Thietan-3-yl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dioxan-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 1,3-Dithiolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy:
   aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl- und Hetarylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
besonders bevorzugt Nitro, Halogen wie z.B. Fluor, Chlor oder Brom, C₁-C₆-Halogenalkyl, -OR⁵ oder -SO₂R⁷ wie z.B. Methylsulfonyl, Ethylsulfonyl oder Difluormethylsulfonyl;
insbesondere bevorzugt Nitro, Fluor, Chlor, Brom, Trifluormethyl, Methoxy, Ethoxy, Methylsulfonyl, Ethylsulfonyl oder Difluormethylsulfonyl;
- R²: Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
besonders bevorzugt Wasserstoff, Nitro, Halogen wie z.B. Fluor, Chlor oder Brom, C₁-C₆-Alkyl wie z.B. Methyl oder Ethyl, C₁-C₆-Halogenalkyl, -OR⁵ oder -SO₂R⁷ wie z.B. Methylsulfonyl, Ethylsulfonyl oder Difluormethylsulfonyl;
insbesondere bevorzugt Nitro, Fluor, Chlor, Brom, Methyl, Ethyl, Trifluormethyl, Methoxy, Ethoxy, Methylsulfonyl, Ethylsulfonyl oder Difluormethylsulfonyl;
- R³: Wasserstoff, Cyano, C₁-C₆-Alkyl, C₁-C₆-Halogenalkyl oder -OR⁷;
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR¹⁰, =NOR¹⁰, -OCOR¹⁰, -CO₂R¹⁰, -COSR¹⁰, -CONR⁸R¹⁰, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy oder Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
besonders bevorzugt C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR¹⁰, =NOR¹⁰, -OCOR¹⁰, -CO₂R¹⁰, -COSR¹⁰, -CONR⁸R¹⁰, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl,
Phenoxy, Benzyloxy oder Hetaryloxy, wobei die achte letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
- X: Sauerstoff oder NH;
- n: 0 oder 2
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl; besonders bevorzugt Methyl, Ethyl, Trifluormethyl, Difluormethyl, Methoxyethyl, Allyl oder Propargyl;
- R⁷: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl; besonders bevorzugt Methyl, Ethyl, Trifluormethyl, Difluormethyl, Methoxyethyl, Allyl oder Propargyl;
- R⁸: Wasserstoff oder C₁-C₆-Alkyl;
- R¹⁰: C₁-C₆-Alkyl, C₁-C₆-Halogenalkyl, C₃-C₆-Alkenyl oder C₃-C₆-Alkinyl;
- R¹¹, R¹², R¹⁴, R¹⁶: Wasserstoff oder C₁-C₄-Alkyl; besonders bevorzugt Wasserstoff, Methyl oder Ethyl;
- R¹³: Wasserstoff, C₁-C₄-Alkyl, C₃-C₄-Cycloalkyl, wobei die beiden letztgenannten Gruppen gegebenenfalls einen bis drei der folgenden Substituenten tragen können: Halogen, C₁-C₄-Alkoxy oder C₁-C₄-Alkylthio;
Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithian-2-yl oder 1,3-Dithiolan-2-yl, wobei die sechs letztgenannten Gruppen jeweils einen bis drei C₁-C₄-Alkylreste tragen können;
besonders bevorzugt Wasserstoff, Methyl, Ethyl, Cyclopropyl, Di(methoxy)methyl, Di(ethoxy)methyl, 2-Ethylthiopropyl, Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 5,5-Dimethyl-1,3-dioxan-2-yl, 1,3-Oxathiolan-2-yl, 1,3-Oxathian-2-yl, 1,3-Dithiolan-2-yl, 5,5-Dimethyl-1,3-dithian-2-yl oder 1-Methylthiocyclopropyl;
- R¹⁵: Wasserstoff, C₁-C₄-Alkyl oder C₁-C₄-Alkoxycarbonyl; besonders bevorzugt Wasserstoff, Methyl oder Methoxycarbonyl.

Ebenso kann vorteilhaft in Betracht kommen, daß R¹³ und R¹⁶ eine π-Bindung ausbilden, so daß ein Doppelbindungssystem entsteht.

Die CR¹³R¹⁴-Einheit kann auch vorteilhaft durch C=O ersetzt werden.

Insbesondere bevorzugt sind Verbindungen der Formel Ia (=̂ I wobei R¹ in Position 4 des Phenylringes und R² in Position 2 des Phenylringes gebunden sind).

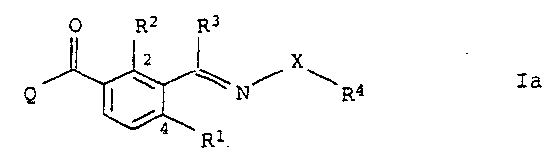

Außerordentlich bevorzugt sind die Verbindungen der Formel Ia in der R¹ bis R³, Q und X die oben genannte Bedeutung haben und
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR¹⁰, =NOR¹⁰, -OCOR¹⁰, -CO₂R¹⁰, -COSR¹⁰, -CONR⁸R¹⁰, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy oder Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
bedeutet.

Insbesondere außerordentlich bevorzugt sind die Verbindungen Ia1 (=̂ I mit R¹ = C1, R¹¹, R¹², R¹³, R¹⁴, R¹⁵, R¹⁶ = H, wobei R¹ in 4-Position des Phenylringes und R² in 2-Position des Phenylringes gebunden sind), insbesondere die Verbindungen der Tabelle 1.

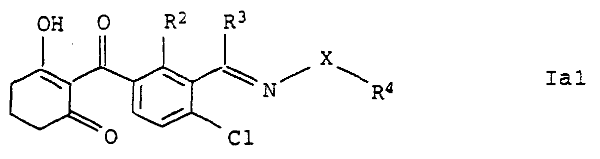

**Tabelle 1**

| Nr. | R² | R³ | R⁴ | X |
|---|---|---|---|---|
| Ia1.001 | Cl | H | CH₃ | O |
| Ia1.002 | Cl | H | C₂H₅ | O |
| Ia1.003 | Cl | H | CH₂-C≡CH | O |
| Ia1.004 | Cl | CH₃ | CH₃ | O |
| Ia1.005 | Cl | CH₃ | C₂H₅ | O |
| Ia1.006 | Cl | CH₃ | CH₂-C≡CH | O |
| Ia1.007 | Cl | C₂H₅ | CH₃ | O |
| Ia1.008 | Cl | C₂H₅ | C₂H₅ | O |
| Ia1.009 | Cl | C₂H₅ | CH₂-C≡CH | O |
| Ia1.010 | Cl | OCH₃ | CH₃ | O |
| Ia1.011 | Cl | OCH₃ | C₂H₅ | O |
| Ia1.012 | Cl | OCH₃ | CH₂-C≡CH | O |
| Ia1.013 | Cl | OC₂H₅ | CH₃ | O |
| Ia1.014 | Cl | OC₂H₅ | C₂H₅ | O |
| Ia1.015 | Cl | OC₂H₅ | CH₂-C≡CH | O |
| Ia1.016 | Cl | H | CH₃ | NH |
| Ia1.017 | Cl | H | C₂H₅ | NH |
| Ia1.018 | Cl | H | CH₂-C≡CH | NH |
| Ia1.019 | Cl | CH₃ | CH₃ | NH |
| Ia1.020 | Cl | CH₃ | C₂H₅ | NH |
| Ia1.021 | Cl | CH₃ | CH₂-C=CH | NH |
| Ia1.022 | Cl | C₂H₅ | CH₃ | NH |
| Ia1.023 | Cl | C₂H₅ | C₂H₅ | NH |
| Ia1.024 | Cl | C₂H₅ | CH₂-C≡CH | NH |
| Ia1.025 | Cl | OCH₃ | CH₃ | NH |
| Ia1.026 | Cl | OCH₃ | C₂H₅ | NH |
| Ia1.027 | Cl | OCH₃ | CH₂-C≡CH | NH |
| Ia1.028 | Cl | OC₂H₅ | CH₃ | NH |
| Ia1.029 | Cl | OC₂H₅ | C₂H₅ | NH |
| Ia1.030 | Cl | OC₂H₅ | CH₂-C≡CH | NH |
| Ia1.031 | CH₃ | H | CH₃ | O |
| Ia1.032 | CH₃ | H | C₂H₅ | O |
| Ia1.033 | CH₃ | H | CH₂-C≡CH | O |
| Ia1.034 | CH₃ | CH₃ | CH₃ | 0 |
| Ia1.035 | CH₃ | CH₃ | C₂H₅ | O |
| Ia1.036 | CH₃ | CH₃ | CH₂-C≡CH | O |
| Ia1.037 | CH₃ | C₂H₅ | CH₃ | O |
| Ia1.038 | CH₃ | C₂H₅ | C₂H₅ | O |
| Ia1.039 | CH₃ | C₂H₅ | CH₂-C≡CH | O |
| Ia1.040 | CH₃ | OCH₃ | CH₃ | O |
| Ia1.041 | CH₃ | OCH₃ | C₂H₅ | O |
| Ia1.042 | CH₃ | OCH₃ | CH₂-C≡CH | O |
| Ia1.043 | CH₃ | OC₂H₅ | CH₃ | O |
| Ia1.044 | CH₃ | OC₂H₅ | C₂H₅ | O |
| Ia1.045 | CH₃ | OC₂H₅ | CH₂-C≡CH | O |
| Ia1.046 | CH₃ | H | CH₃ | NH |
| Ia1.047 | CH₃ | H | C₂H₅ | NH |
| Ia1.048 | CH₃ | H | CH₂-C≡CH | NH |
| Ia1.049 | CH₃ | CH₃ | CH₃ | NH |
| Ia1.050 | CH₃ | CH₃ | C₂H₅ | NH |
| Ia1.051 | CH₃ | CH₃ | CH₂-C≡CH | NH |
| Ia1.052 | CH₃ | C₂H₅ | CH₃ | NH |
| Ia1.053 | CH₃ | C₂H₅ | C₂H₅ | NH |
| Ia1.054 | CH₃ | C₂H₅ | CH₂-C≡CH | NH |
| Ia1.055 | CH₃ | OCH₃ | CH₃ | NH |
| Ia1.056 | CH₃ | OCH₃ | C₂H₅ | NH |
| Ia1.057 | CH₃ | OCH₃ | CH₂-C≡CH | NH |
| Ia1.058 | CH₃ | OC₂H₅ | CH₃ | NH |
| Ia1.059 | CH₃ | OC₂H₅ | C₂H₅ | NH |
| Ia1.060 | CH₃ | OC₂H₅ | CH₂-C≡CH | NH |
| Ia1.061 | OCH₃ | H | CH₃ | O |
| Ia1.062 | OCH₃ | H | C₂H₅ | O |
| Ia1.063 | OCH₃ | H | CH₂-C≡CH | O |
| Ia1.064 | OCH₃ | CH₃ | CH₃ | O |
| Ia1.065 | OCH₃ | CH₃ | C₂H₅ | O |
| Ia1.066 | OCH₃ | CH₃ | CH₂-C≡CH | O |
| Ia1.067 | OCH₃ | C₂H₅ | CH₃ | O |
| Ia1.068 | OCH₃ | C₂H₅ | C₂H₅ | O |
| Ia1.069 | OCH₃ | C₂H₅ | CH₂-C≡CH | O |
| Ia1.070 | OCH₃ | OCH₃ | CH₃ | 0 |
| Ia1.071 | OCH₃ | OCH₃ | C₂H₅ | O |
| Ia1.072 | OCH₃ | OCH₃ | CH₂-C≡CH | O |
| Ia1.073 | OCH₃ | OC₂H₅ | CH₃ | O |
| Ia1.074 | OCH₃ | OC₂H₅ | C₂H₅ | O |
| Ia1.075 | OCH₃ | OC₂H₅ | CH₂-C≡CH | O |
| Ia1.076 | OCH₃ | H | CH₃ | NH |
| Ia1.077 | OCH₃ | H | C₂H₅ | NH |
| Ia1.078 | OCH₃ | H | CH₂-C≡CH | NH |
| Ia1.079 | OCH₃ | CH₃ | CH₃ | NH |
| Ia1.080 | OCH₃ | CH₃ | C₂H₅ | NH |
| Ia1.081 | OCH₃ | CH₃ | CH₂-C≡CH | NH |
| Ia1.082 | OCH₃ | C₂H₅ | CH₃ | NH |
| Ia1.083 | OCH₃ | C₂H₅ | C₂H₅ | NH |
| Ia1.084 | OCH₃ | C₂H₅ | CH₂-C≡CH | NH |
| Ia1.085 | OCH₃ | OCH₃ | CH₃ | NH |
| Ia1.086 | OCH₃ | OCH₃ | C₂H₅ | NH |
| Ia1.087 | OCH₃ | OCH₃ | CH₂-C≡CH | NH |
| Ia1.088 | OCH₃ | OC₂H₅ | CH₃ | NH |
| Ia1.089 | OCH₃ | OC₂H₅ | C₂H₅ | NH |
| Ia1.090 | OCH₃ | OC₂H₅ | CH₂-C≡CH | NH |
| Ia1.091 | CF₃ | H | CH₃ | O |
| Ia1.092 | CF₃ | H | C₂H₅ | O |
| Ia1.093 | CF₃ | H | CH₂-C≡CH | O |
| Ia1.094 | CF₃ | CH₃ | CH₃ | O |
| Ia1.095 | CF₃ | CH₃ | C₂H₅ | O |
| Ia1.096 | CF₃ | CH₃ | CH₂-C≡CH | O |
| Ia1.097 | CF₃ | C₂H₅ | CH₃ | O |
| Ia1.098 | CF₃ | C₂H₅ | C₂H₅ | O |
| Ia1.099 | CF₃ | C₂H₅ | CH₂-C≡CH | O |
| Ia1.100 | CF₃ | OCH₃ | CH₃ | O |
| Ia1.101 | CF₃ | OCH₃ | C₂H₅ | O |
| Ia1.102 | CF₃ | OCH₃ | CH₂-C≡CH | O |
| Ia1.103 | CF₃ | OC₂H₅ | CH₃ | O |
| Ia1.104 | CF₃ | OC₂H₅ | C₂H₅ | O |
| Ia1.105 | CF₃ | OC₂H₅ | CH₂-C≡CH | O |
| Ia1.106 | CF₃ | H | CH₃ | NH |
| Ia1.107 | CF₃ | H | C₂H₅ | NH |
| Ia1.108 | CF₃ | H | CH₂-C≡CH | NH |
| Ia1.109 | CF₃ | CH₃ | CH₃ | NH |
| Ia1.110 | CF₃ | CH₃ | C₂H₅ | NH |
| Ia1.111 | CF₃ | CH₃ | CH₂-C≡CH | NH |
| Ia1.112 | CF₃ | C₂H₅ | CH₃ | NH |
| Ia1.113 | CF₃ | C₂H₅ | C₂H₅ | NH |
| Ia1.114 | CF₃ | C₂H₅ | CH₂-C≡CH | NH |
| Ia1.115 | CF₃ | OCH₃ | CH₃ | NH |
| Ia1.116 | CF₃ | OCH₃ | C₂H₅ | NH |
| Ia1.117 | CF₃ | OCH₃ | CH₂-C≡CH | NH |
| Ia1.118 | CF₃ | OC₂H₅ | CH₃ | NH |
| Ia1.119 | CF₃ | OC₂H₅ | C₂H₅ | NH |
| Ia1.120 | CF₃ | OC₂H₅ | CH₂-C≡CH | NH |
| Ia1.121 | SO₂CH₃ | H | CH₃ | O |
| Ial.122 | SO₂CH₃ | H | C₂H₅ | O |
| Ia1.123 | SO₂CH₃ | H | CH₂-C≡CH | O |
| Ia1.124 | SO₂CH₃ | CH₃ | CH₃ | O |
| Ia1.125 | SO₂CH₃ | CH₃ | C₂H₅ | O |
| Ia1.126 | SO₂CH₃ | CH₃ | CH₂-C≡CH | O |
| Ia1.127 | SO₂CH₃ | C₂H₅ | CH₃ | O |
| Ia1.128 | SO₂CH₃ | C₂H₅ | C₂H₅ | O |
| Ia1.129 | SO₂CH₃ | C₂H₅ | CH₂-C≡CH | O |
| Ia1.130 | SO₂CH₃ | OCH₃ | CH₃ | O |
| Ia1.131 | SO₂CH₃ | OCH₃ | C₂H₅ | O |
| Ia1.132 | SO₂CH₃ | OCH₃ | CH₂-C≡CH | O |
| Ia1.133 | SO₂CH₃ | OC₂H₅ | CH₃ | O |
| Ia1.134 | SO₂CH₃ | OC₂H₅ | C₂H₅ | O |
| Ia1.135 | SO₂CH₃ | OC₂H₅ | CH₂-C≡CH | O |
| Ia1.136 | SO₂CH₃ | H | CH₃ | NH |
| Ia1.137 | SO₂CH₃ | H | C₂H₅ | NH |
| Ia1.138 | SO₂CH₃ | H | CH₂-C≡CH | NH |
| Ia1.139 | SO₂CH₃ | CH₃ | CH₃ | NH |
| Ia1.140 | SO₂CH₃ | CH₃ | C₂H₅ | NH |
| Ia1.141 | SO₂CH₃ | CH₃ | CH₂-C≡CH | NH |
| Ia1.142 | SO₂CH₃ | C₂H₅ | CH₃ | NH |
| Ia1.143 | SO₂CH₃ | C₂H₅ | C₂H₅ | NH |
| Ia1.144 | SO₂CH₃ | C₂H₅ | CH₂-C≡CH | NH |
| Ia1.145 | SO₂CH₃ | OCH₃ | CH₃ | NH |
| Ia1.146 | SO₂CH₃ | OCH₃ | C₂H₅ | NH |
| Ia1.147 | SO₂CH₃ | OCH₃ | CH₂-C≡CH | NH |
| Ia1.148 | SO₂CH₃ | OC₂H₅ | CH₃ | NH |
| Ia1.149 | SO₂CH₃ | OC₂H₅ | C₂H₅ | NH |
| Ia1.150 | SO₂CH₃ | OC₂H₅ | CH₂-C≡CH | NH |
| Ia1.151 | NO₂ | H | CH₃ | O |
| Ia1.152 | NO₂ | H | C₂H₅ | O |
| Ia1.153 | NO₂ | H | CH₂-C≡CH | O |
| Ia1.154 | NO₂ | CH₃ | CH₃ | O |
| Ia1.155 | NO₂ | CH₃ | C₂H₅ | O |
| Ia1.156 | NO₂ | CH₃ | CH₂-C≡CH | O |
| Ia1.157 | NO₂ | C₂H₅ | CH₃ | O |
| Ia1.158 | NO₂ | C₂H₅ | C₂H₅ | O |
| Ia1.159 | NO₂ | C₂H₅ | CH₂-C≡CH | O |
| Ia1.160 | NO₂ | OCH₃ | CH₃ | O |
| Ia1.161 | NO₂ | OCH₃ | C₂H₅ | O |
| Ia1.162 | NO₂ | OCH₃ | CH₂-C≡CH | O |
| Ia1.163 | NO₂ | OC₂H₅ | CH₃ | O |
| Ia1.164 | NO₂ | OC₂H₅ | C₂H₅ | O |
| Ia1.165 | NO₂ | OC₂H₅ | CH₂-C≡CH | O |
| Ia1.166 | NO₂ | H | CH₃ | NH |
| Ia1.167 | NO₂ | H | C₂H₅ | NH |
| Ia1.168 | NO₂ | H | CH₂-C≡CH | NH |
| Ia1.169 | NO₂ | CH₃ | CH₃ | NH |
| Ia1.170 | NO₂ | CH₃ | C₂H₅ | NH |
| Ia1.171 | NO₂ | CH₃ | CH₂-C≡CH | NH |
| Ia1.172 | NO₂ | C₂H₅ | CH₃ | NH |
| Ia1.173 | NO₂ | C₂H₅ | C₂H₅ | NH |
| Ia1.174 | NO₂ | C₂H₅ | CH₂-C≡CH | NH |
| Ia1.175 | NO₂ | OCH₃ | CH₃ | NH |
| Ia1.176 | NO₂ | OCH₃ | C₂H₅ | NH |
| Ia1.177 | NO₂ | OCH₃ | CH₂-C≡CH | NH |
| Ia1.178 | NO₂ | OC₂H₅ | CH₃ | NH |
| Ia1.179 | NO₂ | OC₂H₅ | C₂H₅ | NH |
| Ia1.180 | NO₂ | OC₂H₅ | CH₂-C≡CH | NH |

Des weiteren sind die folgenden 2-Benzoyl-cyclohexan-1,3-dione der Formel I insbesondere außerordentlich bevorzugt:
- die Verbindungen Ia2, insbesondere die Verbindungen Ia2.001-Ia2.180, die sich von den entsprechenden Verbindungen Ia1.001-Ia1.180 dadurch unterscheiden, daß R¹³ für Methyl steht: 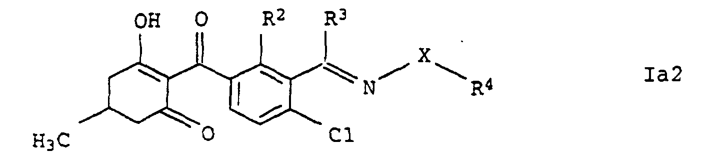
- die Verbindungen Ia3, insbesondere die Verbindungen Ia3.001-Ia3.180, die sich von den entsprechenden Verbindungen Ia1.001-Ia1.180 dadurch unterscheiden, daß R¹³ und R¹⁴ jeweils für Methyl stehen: 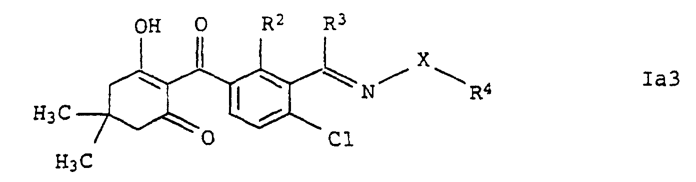
- die Verbindungen Ia4, insbesondere die Verbindungen Ia4.001-Ia4.180, die sich von den entsprechenden Verbindungen Ia1.001-Ia1.180 dadurch unterscheiden, daß R¹⁵ und R¹⁶ jeweils für Methyl stehen: 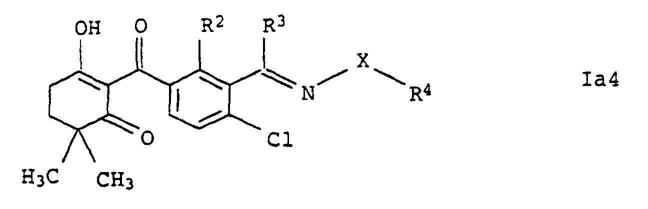
- die Verbindungen Ia5, insbesondere die Verbindungen Ia5.001-Ia5.180, die sich von den entsprechenden Verbindungen Ia1.001-Ia1.180 dadurch unterscheiden, daß die CR¹³R¹⁴-Einheit durch C=O ersetzt ist: 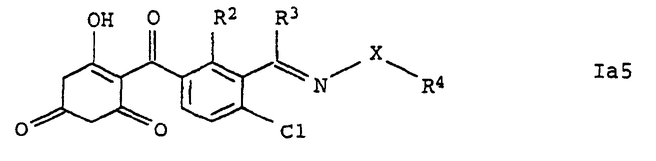
- die Verbindungen Ia6, insbesondere die Verbindungen Ia6.001-Ia6.180, die sich von den entsprechenden Verbindungen Ia1.001-Ia1.180 dadurch unterscheiden, daß R¹¹, R¹⁵ und R¹⁶ jeweils für Methyl stehen und die CR¹³R¹⁴-Einheit durch C=O ersetzt ist: 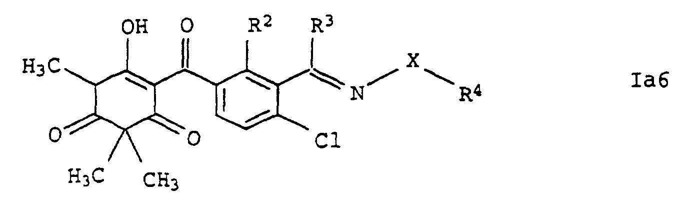
- die Verbindungen Ia7, insbesondere die Verbindungen Ia7.001-Ia7.180, die sich von den entsprechenden Verbindungen Ia1.001-Ia1.180 dadurch unterscheiden, daß R¹¹, R¹², R¹⁵ und R¹⁶ jeweils für Methyl stehen und die CR¹³R¹⁴-Einheit durch C=O ersetzt ist: 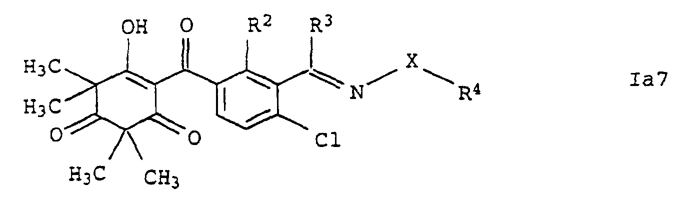
- die Verbindungen Ia8, insbesondere die Verbindungen Ia8.001-Ia8.180, die sich von den entsprechenden Verbindungen Ia1.001-Ia1.180 dadurch unterscheiden, daß R¹ für Nitro steht: 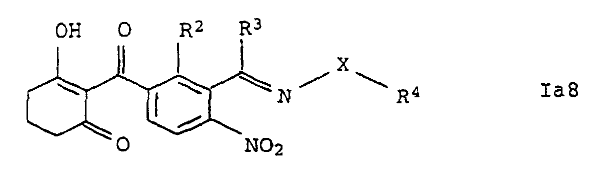
- die Verbindungen Ia9, insbesondere die Verbindungen Ia9.001-Ia9.180, die sich von den entsprechenden Verbindungen Ia1.001-Ia1.180 dadurch unterscheiden, daß R¹ für Nitro und R¹³ für Methyl stehen: 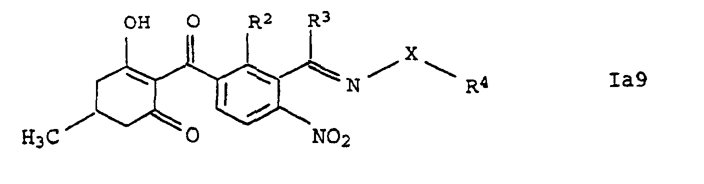
- die Verbindungen Ia10, insbesondere die Verbindungen Ia10.001-Ia10.180, die sich von den entsprechenden Verbindungen Ia1.001-Ia1.180 dadurch unterscheiden, daß R¹ für Nitro und R¹³ und R¹⁴ jeweils für Methyl stehen: 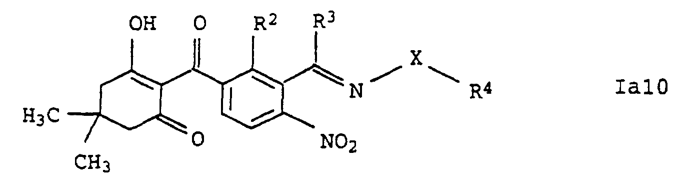
- die Verbindungen Ia11, insbesondere die Verbindungen Ia11.001-Ia11.180, die sich von den entsprechenden Verbindungen Ia1.001-Ia1.180 dadurch unterscheiden, daß R¹ für Nitro und R¹⁵ und R¹⁶ jeweils für Methyl stehen: 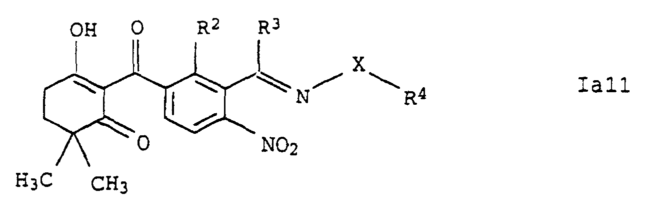
- die Verbindungen Ia12, insbesondere die Verbindungen Ia12.001-Ia12.180, die sich von den entsprechenden Verbindungen Ia1.001-Ia1.180 dadurch unterscheiden, daß R¹ für Nitro steht und die CR¹³R¹⁴-Einheit durch C=O ersetzt ist: 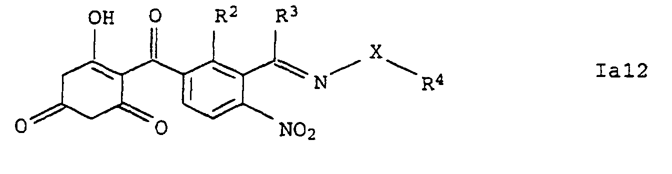
- die Verbindungen Ia13, insbesondere die Verbindungen Ia13.001-Ia13.180, die sich von den entsprechenden Verbindungen Ia1.001-Ia1.180 dadurch unterscheiden, daß R¹ für Nitro, R¹¹, R¹⁵ und R¹⁶ jeweils für Methyl stehen und die CR¹³R¹⁴-Einheit durch C=O ersetzt ist: 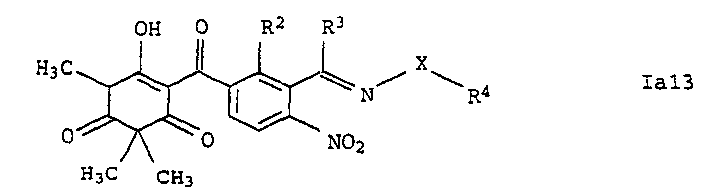
- die Verbindungen Ia14, insbesondere die Verbindungen Ia14.001-Ia14.180, die sich von den entsprechenden Verbindungen Ia1.001-Ia1.180 dadurch unterscheiden, daß R¹ für Nitro, R¹¹, R¹², R¹⁵ und R¹⁶ jeweils für Methyl stehen und die CR¹³R¹⁴-Einheit durch C=O ersetzt ist: 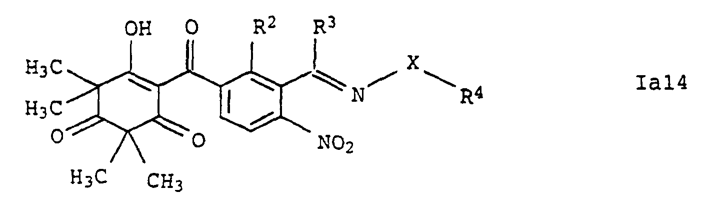
- die Verbindungen Ia15, insbesondere die Verbindungen Ia15.001-Ia15.180, die sich von den entsprechenden Verbindungen Ia1.001-Ia1.180 dadurch unterscheiden, daß R¹ für Methylsulfonyl steht: 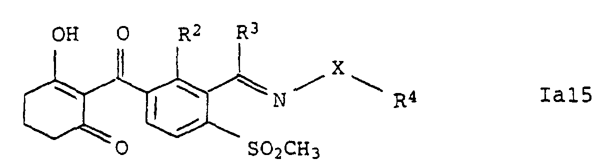
- die Verbindungen Ia16, insbesondere die Verbindungen Ia16.001-Ia16.180, die sich von den entsprechenden Verbindungen Ia1.001-Ia1.180 dadurch unterscheiden, daß R¹ für Methylsulfonyl und R¹³ für Methyl stehen: 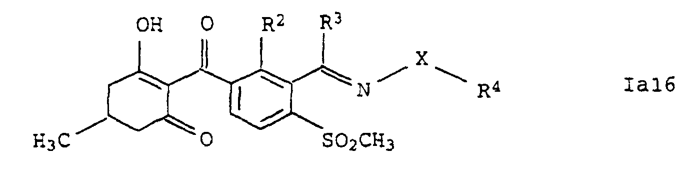
- die Verbindungen Ia17, insbesondere die Verbindungen Ia17.001-Ia17.180, die sich von den entsprechenden Verbindungen Ia1.001-Ia1.180 dadurch unterscheiden, daß R¹ für Methylsulfonyl und R¹³ und R¹⁴ jeweils für Methyl stehen: 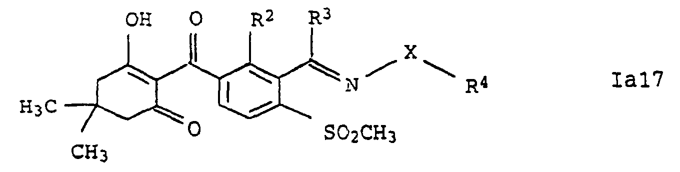
- die Verbindungen Ia18, insbesondere die Verbindungen Ia18.001-Ia18.180, die sich von den entsprechenden Verbindungen Ia1.001-Ia1.180 dadurch unterscheiden, daß R¹ für Methylsulfonyl und R¹⁵ und R¹⁶ jeweils für Methyl stehen: 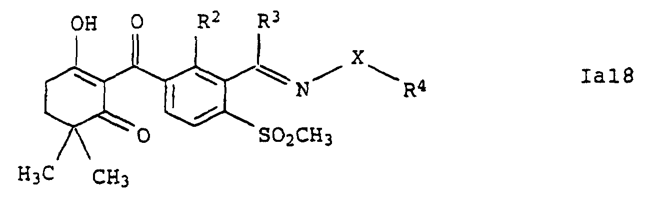
- die Verbindungen Ia19, insbesondere die Verbindungen Ia19.001-Ia19.180, die sich von den entsprechenden Verbindungen Ia1.001-Ia1.180 dadurch unterscheiden, daß R¹ für Methylsulfonyl steht und die CR¹³R¹⁴-Einheit durch C=O ersetzt ist: 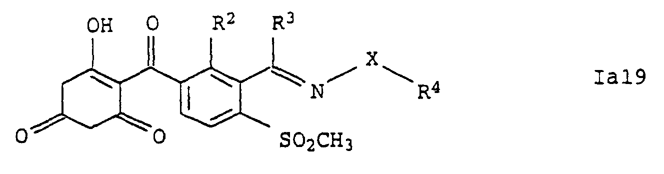
- die Verbindungen Ia20, insbesondere die Verbindungen Ia20.001-Ia20.180, die sich von den entsprechenden Verbindungen Ia1.001-Ia1.180 dadurch unterscheiden, daß R¹ für Methylsulfonyl, R¹¹, R¹⁵ und R¹⁶ jeweils für Methyl stehen und die CR¹³R¹⁴-Einheit durch C=O ersetzt ist: 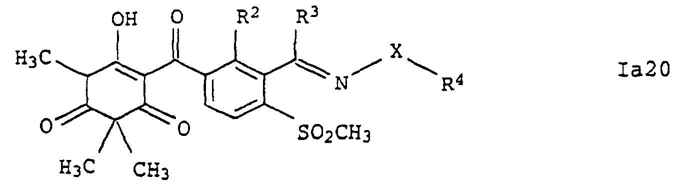
- die Verbindungen Ia21, insbesondere die Verbindungen Ia21.001-Ia21.180, die sich von den entsprechenden Verbindungen Ia1.001-Ia1.180 dadurch unterscheiden, daß R¹ für Methylsulfonyl, R¹¹, R¹², R¹⁵ und R¹⁶ jeweils für Methyl stehen und die CR¹³R¹⁴-Einheit durch C=O ersetzt ist: 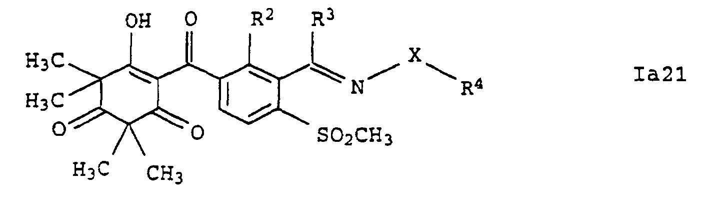
- die Verbindungen Ia22, insbesondere die Verbindungen Ia22.001-Ia22.180, die sich von den entsprechenden Verbindungen Ia1.001-Ia1.180 dadurch unterscheiden, daß R¹ für Trifluormethyl steht: 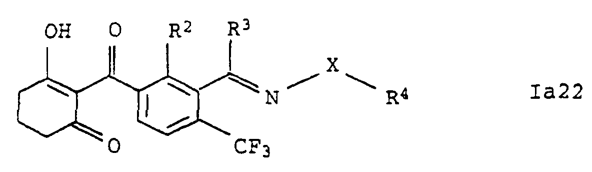
- die Verbindungen Ia23, insbesondere die Verbindungen Ia23.001-Ia23.180, die sich von den entsprechenden Verbindungen Ia1.001-Ia1.180 dadurch unterscheiden, daß R¹ für Trifluormethyl und R¹³ für Methyl stehen: 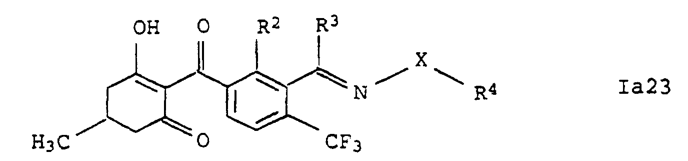
- die Verbindungen Ia24, insbesondere die Verbindungen Ia24.001-Ia24.180, die sich von den entsprechenden Verbindungen Ia1.001-Ia1.180 dadurch unterscheiden, daß R¹ für Trifluormethyl und R¹³ und R¹⁴ jeweils für Methyl stehen: 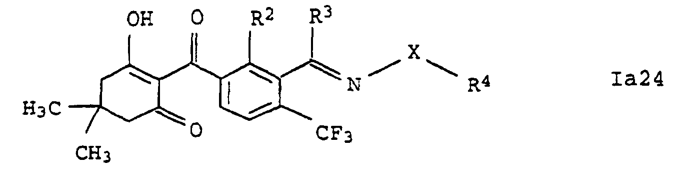
- die Verbindungen Ia25, insbesondere die Verbindungen Ia25.001-Ia25.180, die sich von den entsprechenden Verbindungen Ia1.001-Ia1.180 dadurch unterscheiden, daß R¹ für Trifluormethyl und R¹⁵ und R¹⁶ jeweils für Methyl stehen: 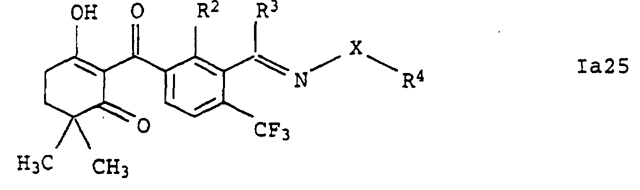
- die Verbindungen Ia26, insbesondere die Verbindungen Ia26.001-Ia26.180, die sich von den entsprechenden Verbindungen Ia1.001-Ia1.180 dadurch unterscheiden, daß R¹ für Trifluormethyl steht und die CR¹³R¹⁴-Einheit durch C=O ersetzt ist: 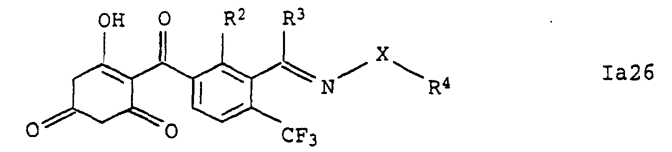
- die Verbindungen Ia27, insbesondere die Verbindungen Ia27.001-Ia27.180, die sich von den entsprechenden Verbindungen Ia1.001-Ia1.180 dadurch unterscheiden, daß R¹ für Trifluormethyl, R¹¹, R¹⁵ und R¹⁶ jeweils für Methyl stehen und die CR¹³R¹⁴-Einheit durch C=O ersetzt ist: 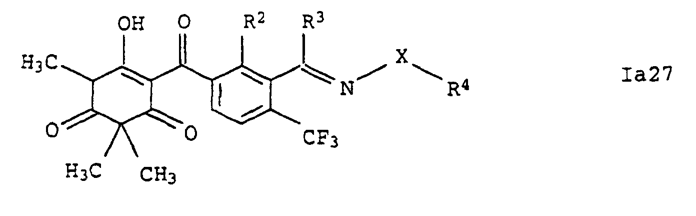
- die Verbindungen Ia28, insbesondere die Verbindungen Ia28.001-Ia28.180, die sich von den entsprechenden Verbindungen Ia1.001-Ia1.180 dadurch unterscheiden, daß R¹ für Trifluormethyl, R¹¹, R¹², R¹⁵ und R¹⁶ jeweils für Methyl stehen und die CR¹³R¹⁴-Einheit durch C=O ersetzt ist:

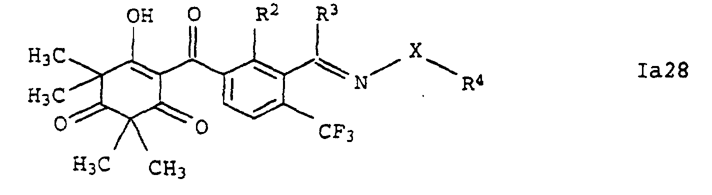

Ganz insbesonderst außerordentlich bevorzugt sind die Verbindungen der Formel Ia' (=̂ I, wobei R¹ in Position 4 des Phenylrings und R² in Position 2 des Phenylrings gebunden sind), 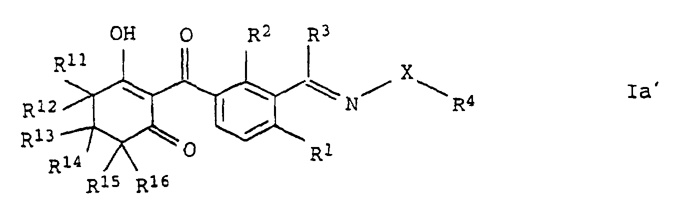 wobei die Variablen folgende Bedeutung haben:
- R¹: Halogen oder C₁-C₄-Alkylsulfonyl;
- R²: Halogen oder C₁-C₄-Alkyl, insbesondere Halogen;
- R³: Wasserstoff, C₁-C₄-Alkyl oder C₁-C₄-Alkoxyl insbesondere Wasserstoff oder C₁-C₄-Alkoxy;
- R⁴: C₁-C₆-Alkyl, C₃-C₆-Alkinyl, wobei diese zwei Substituenten partiell oder vollständig halogeniert sein können und/oder eine bis drei der folgenden Gruppen tragen können:
Phenyl oder Hetaryl, wobei diese ihrerseits partiell oder vollständig halogeniert sein können;
- X: Sauerstoff;
- R¹¹, R¹², R¹³, R¹⁴, R¹⁵, R¹⁶: Wasserstoff oder C₁-C₄-Alkyl;

Die 2-Benzoyl-cyclohexan-1,3-dione der Formel I sind auf verschiedene Art und Weise erhältlich, beispielsweise nach folgendem Verfahren:
Umsetzung von Cyclohexandionen der Formel II mit einer aktivierten Carbonsäure IIIα oder einer Carbonsäure IIIβ, die vorzugsweise in situ aktiviert wird, zu dem Acylierungsprodukt IV und anschließende Umlagerung.

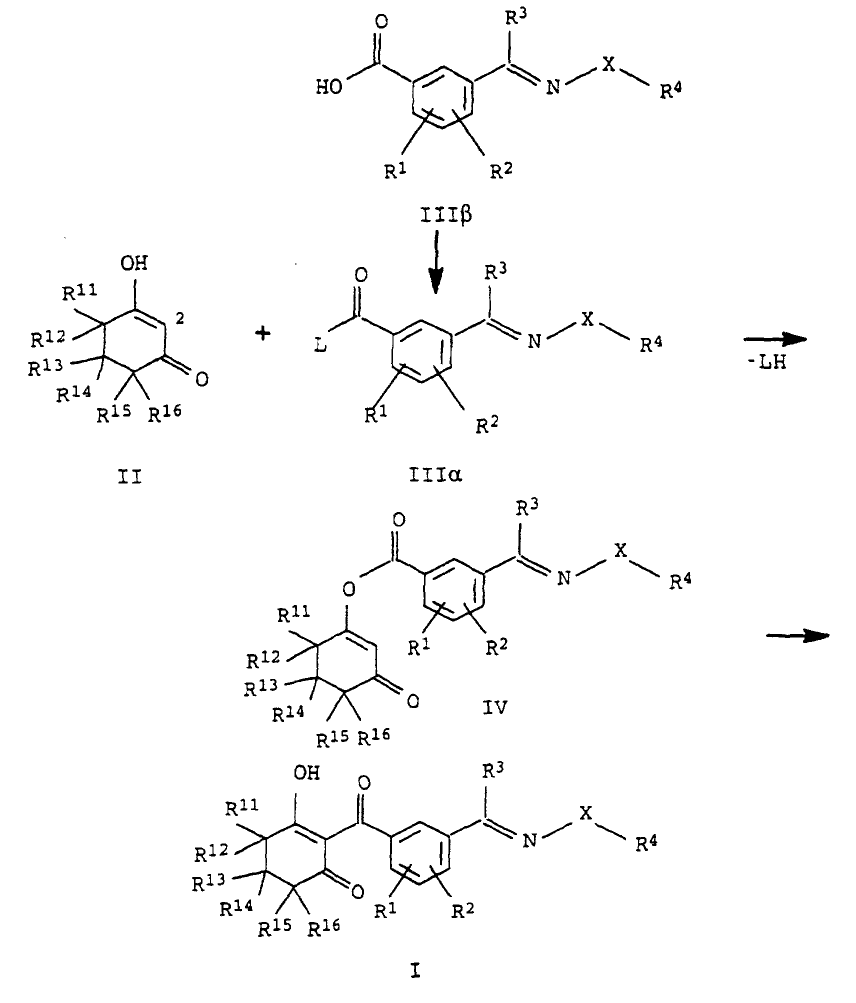

L steht für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolester der Formel IV zu den Verbindungen der Formel I erfolgt zweckmäßigerweise bei Temperaturen von 20 bis 40°C in einem Lösungsmittel und in Gegenwart einer Hilfsbase sowie gegebenenfalls mit Hilfe einer Cyanoverbindung als Katalysator.

Als Lösungsmittel können z.B. Acetonitril, Methylenchlorid, 1,2-Dichlorethan, Essigsäureethylester, Toluol oder Gemische hiervon verwendet werden. Bevorzugtes Lösungsmittel ist Acetonitril.

Geeignete Hilfsbasen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Enolester, eingesetzt werden. Bevorzugt wird Triethylamin verwendet, vorzugsweise in doppelt äquimolaren Verhältnis in Bezug auf den Enolester.

Als "Umlagerungskatalysator" kommen anorganische Cyanide, wie Natriumcyanid, Kaliumcyanid und organische Cyanoverbindungen, wie Acetoncyanhydrin, Trimethylsilylcyanid in Betracht. Sie werden üblicherweise in einer Menge von 1 bis 50 Molprozent, bezogen auf den Enolester, eingesetzt. Vorzugsweise werden Acetoncyanhydrin oder Trimethylsilylcyanid, z.B. in einer Menge von 5 bis 15, vorzugsweise 10 Molprozent, bezogen auf den Enolester, eingesetzt.

Die Aufarbeitung kann in an sich bekannter Weise erfolgen. Das Reaktionsgemisch wird z.B. mit verdünnter Mineralsäure, wie z.B. 5%ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Enolestern von Cyclohexan-1,3-dionen und für die cyanidkatalysierte Umlagerung der Enolester sind z.B. in EP-A 186 118, US 4 780 127 genannt).

Die als Ausgangsmaterialien verwendeten Cyclohexan-1,3-dione der Formel II sind bekannt oder können nach an sich bekannten Verfahren hergestellt werden (z.B. EP-A 71 707, EP-A 142 741, EP-A 243 313, US 4 249 937; WO 92/13821).

Benzoesäurederivate der Formel IIIa sind neu, 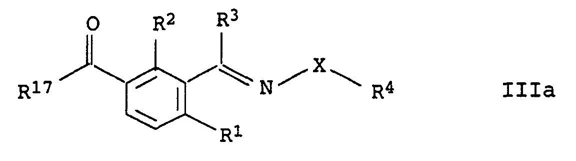 wobei die Variablen folgende Bedeutung haben:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, oder -S(O)ₙR⁷;
- R²: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
- R³: Wasserstoff, Cyano, C₁-C₆-Alkyl, C₁-C₆-Halogenalkyl, -OR⁷, -SR⁷ oder -NR⁷R¹⁰;
- R⁴: C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder-CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits gegebenenfalls substituiert sein können;
- X: Sauerstoff oder NR⁸;
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R6: C₁-C₆-Alkyl oder C₁-C₆-Halogenalkyl;
- R⁷: C₁-C₆-Alkyl, C₁-C₆-Halogenalkyl, C₄-C₆-Alkoxy-C₂-C₆-alkyl, C₃-C₆-Alkenyl oder C₃-C₆-Alkinyl;
- R⁸: Wasserstoff oder C₁-C₆-Alkyl;
- R⁹: C₁-C₆-Alkyl, C₃-C₆-Alkenyl, C₃-C₆-Alkinyl, Phenyl oder Benzyl;
- R¹⁰: C₁-C₆-Alkyl, C₁-C₆-Halogenalkyl, C₃-C₆-Alkenyl oder C₃-C₆-Alkinyl;
- R¹⁷: Hydroxy oder ein abhydrolysierbarer Rest.

Beispiele für abhydrolysierbare Reste sind Alkoxy-, Phenoxy-, Alkylthio-, Phenylthio-Reste, die gegebenenfalls substituiert sein können, Halogenide, Hetarylreste, die über Stickstoff gebunden sind, Amino-, Imino-Reste, die gegebenenfalls substituiert sein können etc.

Bevorzugt sind Benzoesäurehalogenide IIIaα, mit L = Halogen (=̂ IIIa mit R¹⁷ = Halogen), 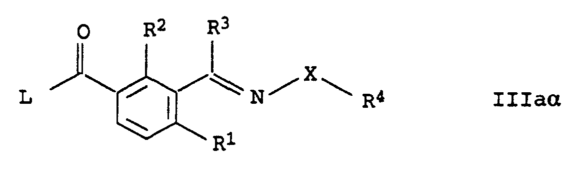 wobei die Variablen R¹ bis R⁴ und X die unter Formel IIIa genannte Bedeutung haben und
- L: Halogen, insbesondere Chlorid oder Bromid, bedeutet.

Ebenso bevorzugt sind die Benzoesäuren der Formel IIIaß (=̂ IIIa mit R¹⁷ = Hydroxy), 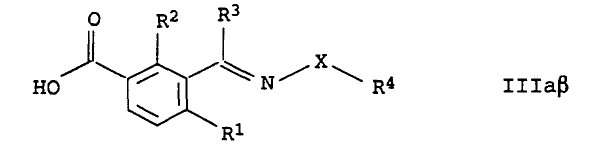 wobei die Variablen R¹ bis R⁴ und X die unter Formel IIIa genannte Bedeutung haben.

Ebenso bevorzugt sind die Benzoesäureester der Formel IIIaγ (=̂ IIIa mit M = C₁-C₆-Alkoxy), 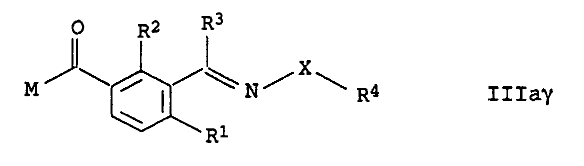 wobei die Variablen R¹ bis R⁴ und X die unter Formel IIIa genannte Bedeutung haben und
- M: C₁-C₆-Alkoxy;
bedeutet.

Im Hinblick auf die bevorzugten Verbindungen der Formel IIIa gelten für die Reste R¹ bis R⁴ und X die unter den Verbindungen I gemachten Ausführungen.

Die Verbindungen der Formel IIIα (mit L = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIß mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIβ können in Analogie zu an sich literaturbekannten Methoden u.a. durch Verseifung der Benzoesäureester der Formel IIIγ (mit M = C₁-C₆-Alkoxy) erhalten werden.

Die Benzoesäureester der Formel IIIγ sind auf verschiedene Art und Weise erhältlich, beispielsweise nach folgenden Verfahren:

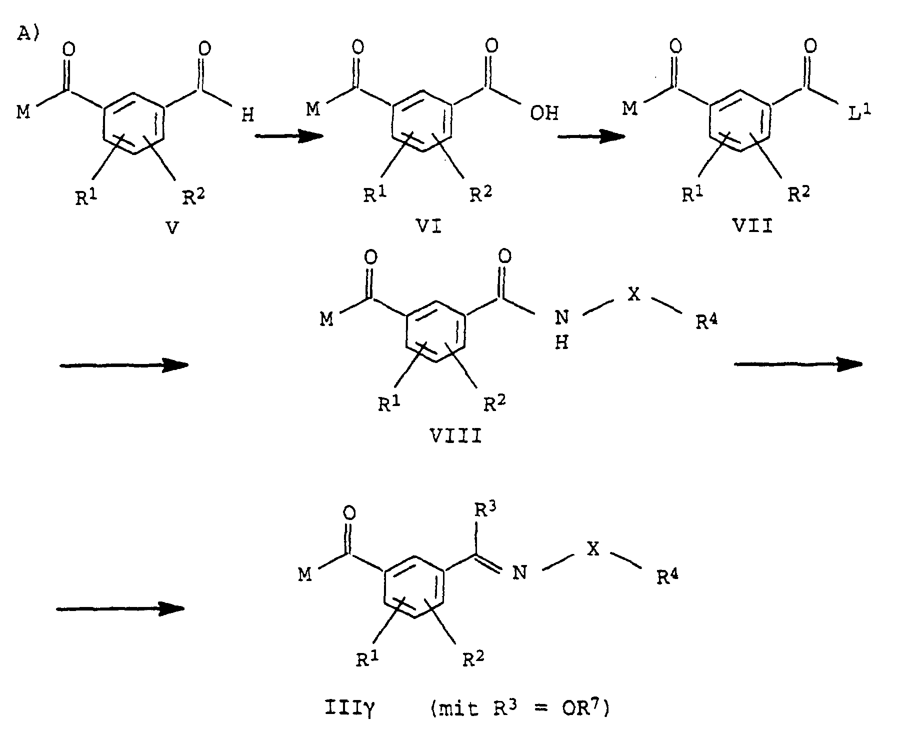

Durch Oxidation von Aldehyden der Formel V können auf an sich bekannte Art und Weise Isophthalsäurederivate der Formel VI erhalten werden (J. March, "Advanced Organic Chemistry", 3. Auflage, S. 629 ff, Wiley-Interscience Publication, 1985).

In Analogie zu literaturbekannten Verfahren können die Verbindungen der Formel VI zunächst in die entsprechenden aktivierten Carbonsäuren VII, wobei L¹ für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat etc. steht, und anschließend in die entsprechenden Hydroxamsäure- bzw. Carbonsäurehydrazidderivate der Formel VIII übergeführt werden (Australian J. Chem. (1969), 22, 1731-1735; ibid (1969), 22, 161-173; J. Org. Chem. (1974), 27, 1341-1349).

Die Alkylierung von Verbindungen der Formel VIII führt zu Verbindungen der Formel IIIγ (mit R³ = OR⁷) auf an sich bekannte Art und Weise (EP-A 463 989; Synthesis (1983), 220-222; US 4 931 088; J. Org. Chem. (1971), 31, 284-294; J. Chem. Soc. Perk. II (1977), 1080-1084).

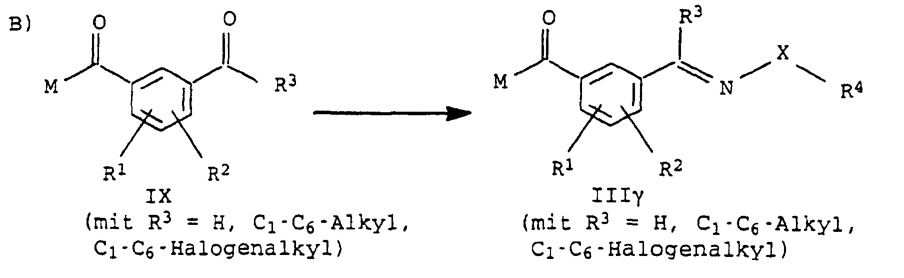

Durch Umsetzung von Aldehyden/Ketonen der Formel IX mit "Alkoxaminen bzw. Alkylhydrazinen" erhält man Verbindungen der Formel IIIγ auf an sich bekannte Weise. In Analogie zu literaturbekannten Verfahren ist es möglich, Aldehyde/Ketone der Formel IX mit Hydroxylamin bzw. Hydrazin umzusetzen und anschließend zu alkylieren (J. March, "Advanced Organic Chemistry", 3. Auflage, S. 359, S. 805-806, Wiley-Interscience Publication, 1985).

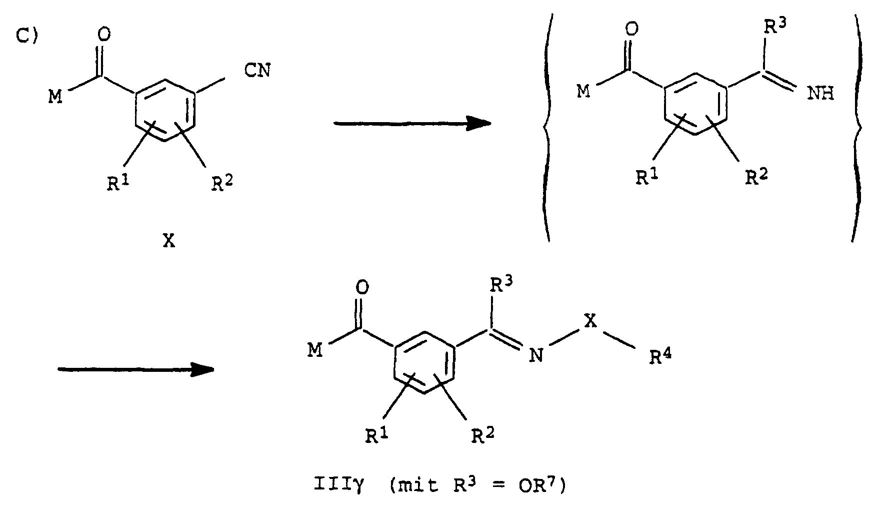

Auf an sich bekannte Weise können Nitrile der Formel X durch Alkoholyse (R⁷OH) in Iminoester übergeführt werden, die in einem weiteren Schritt mit Hydroxylaminen bzw. Hydrazinen zu Verbindungen der Formel IIIγ umgesetzt werden können (J. March, "Advanced Organic Chemistry", 3. Auflage, S. 792-793, Wiley-Interscience Publication, 1985; US 4 965 390).

Die Nitrile der Formel X können in Analogie zu literaturbekannten Verfahren aus den entsprechenden Aldehyden V dargestellt werden (J. March, "Advanced Organic Chemistry", 3. Auflage, S. 806-807, Wiley-Interscience Publication, 1985). Ebenso ist es möglich, Nitrile der Formel X aus Anilinen der Formel XI mittels Sandmeyer-Reaktion zu erhalten oder durch Rosemund/von Braun-Reaktion aus Arylhalogeniden der Formel XII mit Metallcyaniden, insbesondere CuCN (J. March, "Advanced Organic Chemistry", 3. Auflage, S. 594, S. 648, Wiley-Interscience Publication, 1985).

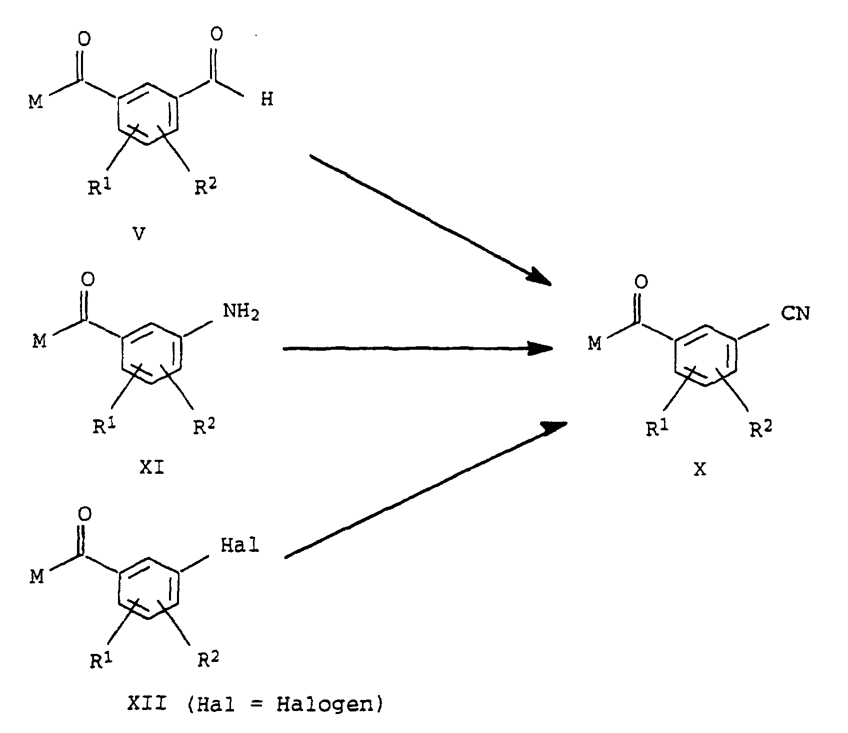

Die Aldehyde der Formel V können in Analogie zu literaturbekannten Verfahren aus entsprechenden Toluolen der Formel XIII dargestellt werden, indem man sie in das ω-Halogentoluol XIV überführt und anschließend oxidiert (vgl. Synth. Commun. 22, 1967-1971 (1992)).

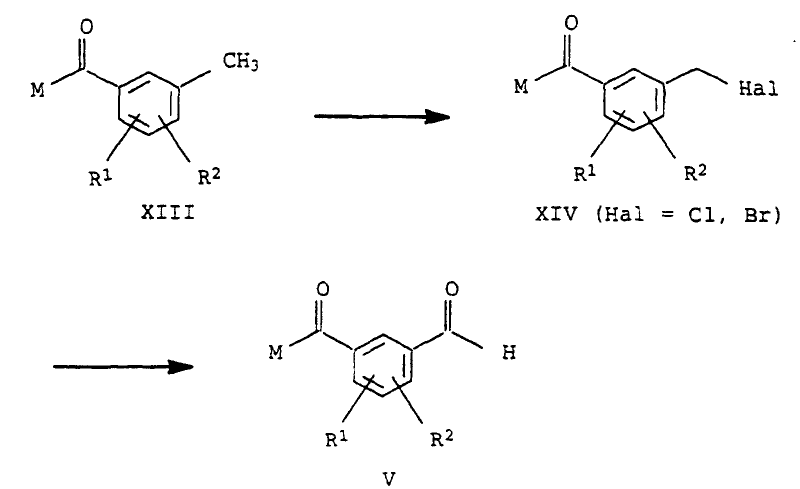

### Herstellungsbeispiele

### 2-(2,4-Dichlor-3-propargyloxyiminomethyl-benzoyl)-5,5-dimethyl-1,3-cyclohexandion (Verbindung 2.12)

Zu einer Lösung von 2,50 g (0,0092 mol) 2,4-Dichlor-3-propargyloxyiminomethyl-benzoesäure in 120 ml trockenem Acetonitril wurden 1,20 g (0,0086 mol) Dimedon und 1,80 g (0,0086 mol) Dicyclohexylcarbodiimid gegeben. Nach 12 Stunden Rühren bei Raumtemperatur wurde über Kieselgel abfiltriert (Eluent: Toluol), anschließend das Lösungsmittel entfernt, der Rückstand in 100 ml trockenem Acetonitril aufgenommen und mit 0,40 g (0,0047 mol) Acetoncyanhydrin und 3,10 g (0,031 mol) Triethylamin versetzt. Nun wurde 3 Stunden bei Raumtemperatur gerührt und dann das Reaktionsgemisch zu einer Mischung aus 200 ml Wasser und 100 ml 5 %ige Kaliumcarbonatlösung gegeben. Die wäßrige Phase wurde dreimal mit Essigsäureethylester gewaschen, anschließend mit 10 %iger Salzsäure auf pH = 2 gestellt und dreimal mit Essigsäureethylester extrahiert. Die vereinigten organischen Phasen wurden mit Wasser neutralgewaschen, getrocknet und eingeengt. Man erhielt 0,7 g 2-(2,4-Dichlor-3-propargyloxyiminomethyl-benzoyl)-5,5-dimethyl-cyclohexandion.
(¹H-NMR (CDCl₃, δ in ppm): 1,13 (6H); 2,32 (2H); 2,52 (1H); 2,66 (2H); 4,81 (2H); 7,12 (1H); 7,43 (1H); 8,31 (1H); 16,48 (1H).)

### 2-(2-Chlor-3-ethoxyiminomethyl-4-methylsulfonyl-benzoyl)-5,5-dimethyl-1,3-cyclohexandion (Verbindung 2.07)

Zu einer Lösung von 2,95 g (0,009 mol) 2-Chlor-3-ethoxyiminomethyl-4-methylsulfonyl-benzoesäure in 130 ml wasserfreiem Acetonitril wurden 1,26 g (0,009 mol) Dimedon und 1,90 g (0,009 mol) Dicyclohexylcarbodiimid gegeben. Nach 12 Stunden Rühren bei Raumtemperatur tropfte man 0,42 g (0,005 mol) Acetoncyanhydrin und 3,27 g (0,032 mol) Triethylamin in 10 ml absolutem Acetonitril zu. Anschließend wurde weitere 2 Stunden bei Raumtemperatur gerührt. Nach Einrühren des Reaktionsgemisches in 200 ml Wasser wurde der Niederschlag abgesaugt und das Filtrat zu 100 ml 5 %iger Kaliumcarbonatlösung gegeben. Nach Waschen der wäßrigen Phase mit Essigsäureethylester wurde mit 10 %iger Salzsäure ein pH-Wert von 2 eingestellt und mit Essigsäureethylester extrahiert. Die vereinigten organischen Phasen wurden mit Wasser neutralgewaschen, getrocknet und am Vakuum eingeengt. Man erhielt 2,52 g Rohprodukt, das aus Toluol umkristallisiert wurde.
(Fp.: 156-157°C)

### 2-[2,4-Dichlor-3-(1'-methoxyimino-1'-(methoxy)methyl)benzoyl]-1,3-cyclohexandion (Verbindung 2.10)

Zu einer Lösung von 0,46 g (0,0045 mol) Triethylamin und 0,5 g (0,0045 mol) 1,3-Cyclohexandion in 50 ml Methylenchlorid wurden 1,125 g (0,0038 mol) 2,4-Dichlor-3(1'-methoxyimino-1'-(methoxy)-methyl)benzoylchlorid gegeben. Nachdem die Reaktionslösung 2 Stunden bei Raumtemperatur gerührt wurde, entfernte man das Lösungsmittel am Vakuum. Der Rückstand wurde mittels Chromatographie an Kieselgel (Eluent: Toluol/Essigsäureethylester = 1/1) gereinigt. Der so erhaltene Enolester wurde in 50 ml Acetonitril aufgenommen und mit 0,80 g (0,008 mol) Triethylamin und 0,15 g (0,0015 mol) Trimethylsilylcyanid versetzt. Nach 12 Stunden Rühren bei Raumtemperatur wurde das Lösungsmittel entfernt und der Rückstand in Methylenchlorid aufgenommen. Die organische Phase wurde mit verdünnter Phoshorsäure gewaschen, getrocknet und eingeengt. Man erhielt 0,90 g 2-[2,4-Dichlor-3-(1'-methoxyimino-1'-(methoxy)methyl)benzoyl]-1,3-cyclohexandion, das aus Diethylether ausgerührt wurde.
(FP: 180-182°C)

In der folgenden Tabelle 2 sind neben den voranstehend beschriebenen Benzoylderivaten der Formel I noch weitere aufgeführt, die in analoger Weise hergestellt wurden oder herstellbar sind:

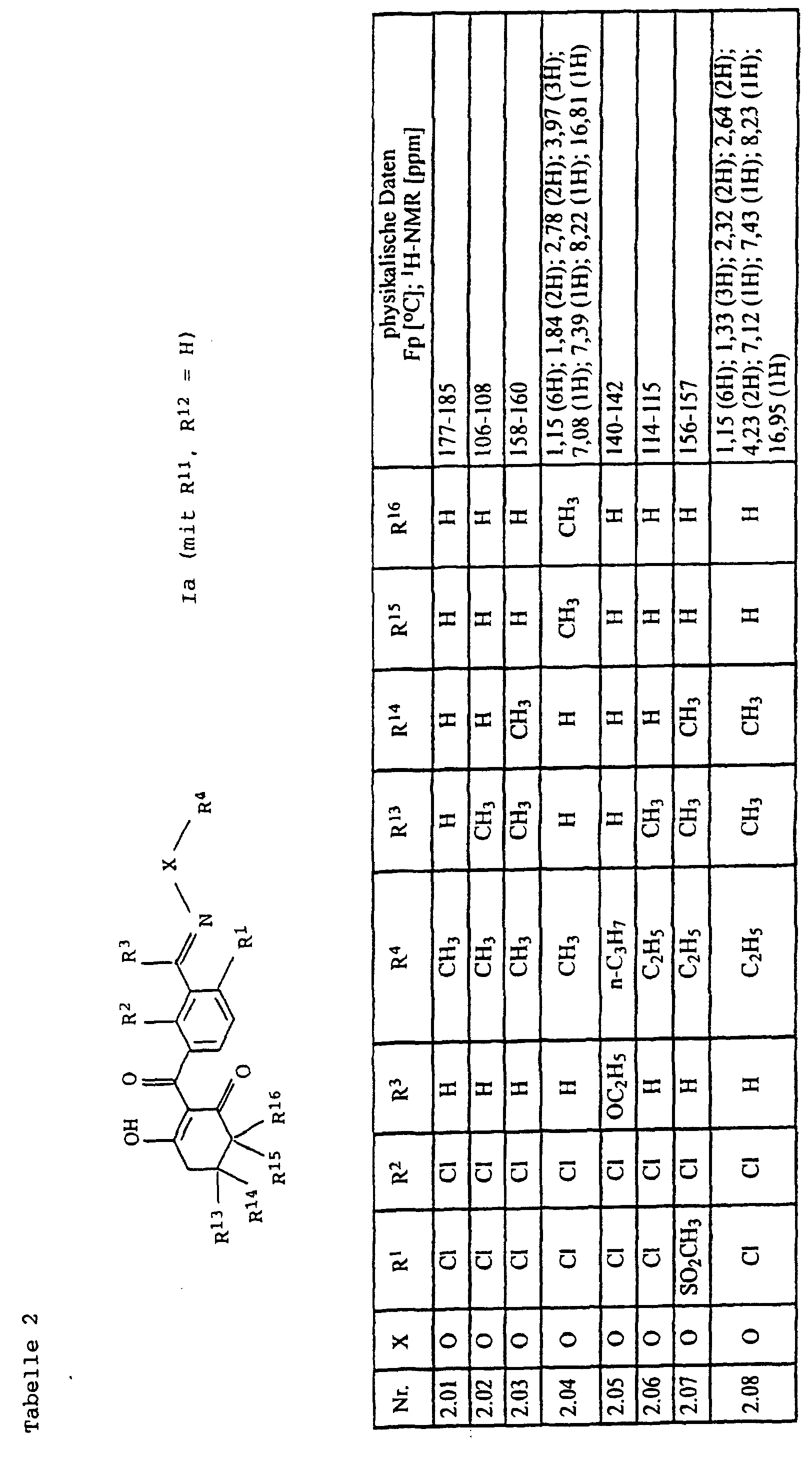

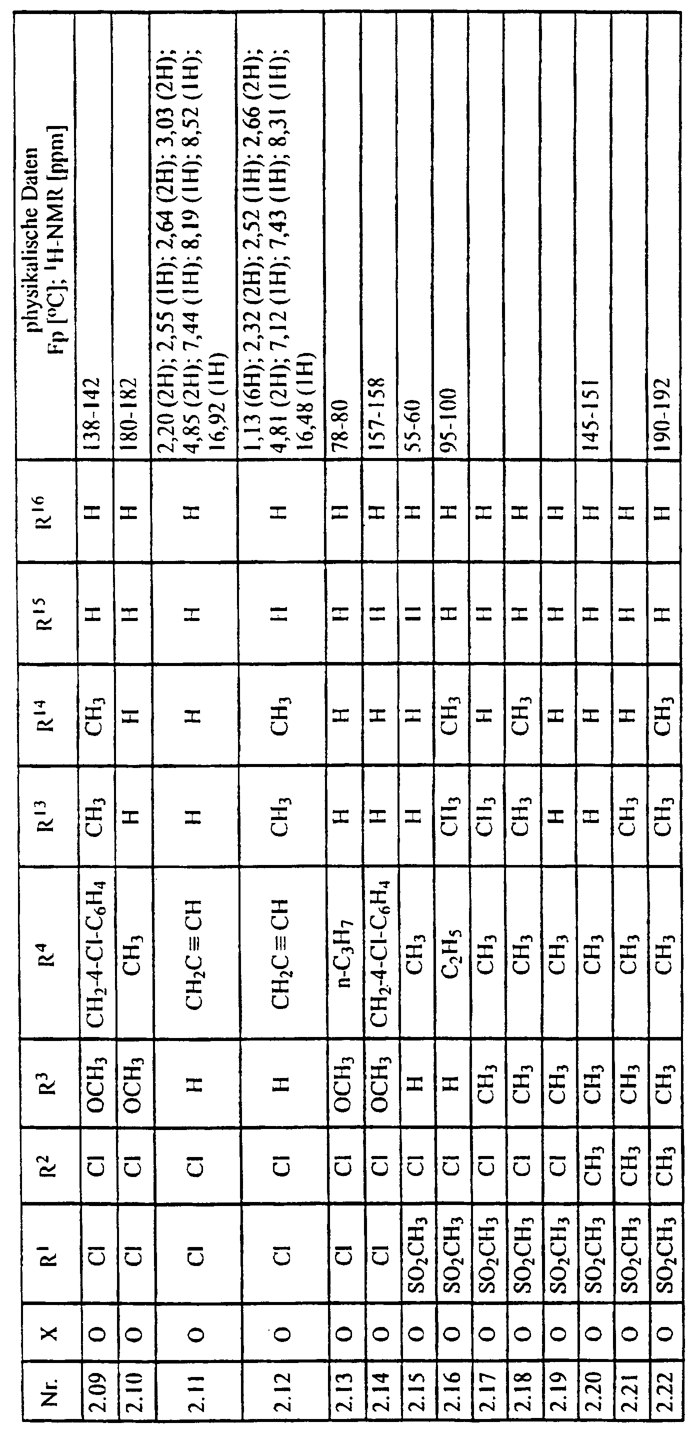

Nachfolgend sind die Synthesen einiger Ausgangsstoffe aufgeführt:

### 2-Chlor-3-ethoxyiminomethyl-4-methylsulfonyl-benzoesäure (Verbindung 3.04)

Stufe a) 2-Chlor-3-methyl-4-methylthio-acetophenon
   Zu einer Suspension von 286 g (2,14 mol) Aluminiumtrichlorid in 420 ml 1,2-Dichlorethan wurde bei 15-20°C eine Lösung von 157 g (2 mol) Acetylchlorid in 420 ml 1,2-Dichlorethan getropft. Anschließend wurde eine Lösung von 346 g (2 mol) 2-Chlor-6-methylthio-toluol in 1 1 1,2-Dichlorethan zugetropft. Nach 12 Stunden Rühren wurde das Reaktionsgemisch in eine Mischung aus 3 1 Eis und 1 1 konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert. Man erhielt 256 g (60 % d.Th.) 2-Chlor-3-methyl-4-methylthio-acetophenon.
   (Fp.: 46°C)
Stufe b) 2-Chlor-3-methyl-4-methylsulfonyl-acetophenon
   163,0 g (0,76 mol) 2-Chlor-3-methyl-4-methylthio-acetophenon wurden in 1,5 1 Eisessig gelöst, mit 18,6 g Natriumwolframat versetzt und unter Kühlung 173,3 g 30 %ige Wasserstoffperoxidlösung zugetropft. Es wurde 2 Tage nachgerührt und anschließend mit wasser verdünnt. Der ausgefallene Feststoff wurde abgesaugt, mit Wasser gewaschen und getrocknet. Man erhielt 164,0 g (88 % d.Th.) 2-Chlor-3-methyl-4-methylsulfonyl-acetophenon.
   (Fp.: 110-111°C)
Stufe c) 2-Chlor-3-methyl-4-methylsulfonyl-benzoesäure
   82 g (0,33 mol) 2-Chlor-3-methyl-4-methylsulfonyl-acetophenon wurden in 700 ml Dioxan gelöst und bei Raumtemperatur mit 1 1 einer 12,5 %igen Natriumhypochloritlösung versetzt. Anschließend wurde 1 Stunde bei 80°C nachgerührt. Nach dem Abkühlen bildeten sich zwei Phasen, von denen die untere mit Wasser verdünnt und schwach angesäuert wurde. Der ausgefallene Feststoff wurde abgesaugt, mit Wasser nachgewaschen und getrocknet. Man erhielt 60 g (73 % d.Th) 2-Chlor-3-methyl-4-methylsulfonylbenzoesäure.
   (Fp.: 230-231°C)
Stufe d) 2-Chlor-3-methyl-4-methylsulfonyl-benzoesäuremethylester
   100 g (0,4 mol) 2-Chlor-3-methyl-4-methylsulfonyl-benzoesäure wurden in 1 1 Methanol gelöst und bei Rückflußtemperatur 5 Stunden mit Chlorwasserstoff begast. Anschließend wurde eingeengt. Man erhielt 88,5 g (84 % d.Th.) 2-Chlor-3-methyl-4-methylsulfonyl-benzoesäuremethylester.
   (Fp.: 107-108°C)
Stufe e) 3-Brommethyl-2-chlor-4-methylsulfonyl-benzoesäuremethylester
   82 g (0,31 mol) 2-Chlor-3-methyl-4-methylsulfonyl-benzoesäuremethylester werden in 2 1 Tetrachlormethan gelöst und unter Belichtung portionsweise mit 56 g (0,31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wurde mit Petrolether versetzt, der ausgefallene Feststoff abgesaugt und getrocknet. Man erhielt 74,5 g (70 % d.Th.) 3-Brommethyl-2-chlor-4-methylsulfonyl-benzoesäuremethylester.
   (Fp.: 74-75°C)
Stufe f) 2-Chlor-3-formyl-4-methylsulfonyl-benzoesäuremethylester
   Eine Lösung von 41,0 g (0,12 mol) 3-Brommethyl-2-chlor-4-methylsulfonyl-benzoesäuremethylester in 250 ml Acetonitril wurde mit 42,1 g (0,36 mol) N-Methylmorpholin-N-oxid versetzt. Der Ansatz wurde 12 Stunden bei Raumtemperatur gerührt, anschließend eingeengt und der Rückstand in Essigsäureethylester aufgenommen. Die Lösung wurde mit Wasser extrahiert, mit Natriumsulfat getrocknet und eingeengt. Man erhielt 31,2 g (94 % d.Th.) 2-Chlor-3-formyl-4-methylsulfonyl-benzoesäuremethylester
   (Fp.: 98-105°C)
Stufe g) 2-Chlor-3-formyl-4-methylsulfonyl-benzoesäure
   Zu einer Lösung von 9,60 g (0,072 mol) Lithiumiodid und 70 ml trockenem Pyridin wurde bei Rückflußtemperatur langsam eine Lösung von 5,00 g (0,018 mol) 2-Chlor-3-formyl-4-methylsulfonyl-benzoesäuremethylester getropft. Nach 2 Stunden Rühren unter Rückfluß kühlte man das Reaktionsgemisch ab und entfernte das Lösungsmittel am Vakuum. Der Rückstand wurde anschließend in Wasser aufgenommen und mit verdünnter Salzsäure auf pH 1-2 eingestellt. Nach Extraktion der wäßrigen Phase mit Essigsäureethylester wurden die gesammelten organischen Phasen mit Wasser neutralgewaschen, getrocknet und eingeengt. Man erhielt 4,00 g 2-Chlor-3-formyl-4-methylsulfonylbenzoesäure (85 % Ausbeute).
   ⁽¹H-NMR (d⁶-DMSO, δ in ppm): 3,41 (s, 3H); 8,05 (d, 1H); 8,11 (d, 1H); 10,49 (s, 1H); 14,21 (s, br., 1H).)
Stufe h) 2-Chlor-3-ethoxyiminomethyl-4-methylsulfonyl-benzoesäure
   1,63 g (0,017 mol) Ethoxyaminhydrochlorid und 1,15 g (0,0085 mol) fein gepulvertes Kaliumcarbonat wurden in 60 ml trockenem Methanol 1 Stunde gerührt. Anschließend gab man 4,00 g (0,015 mol) 2-Chlor-3-formyl-4-methylsulfonyl-benzoesäure in 40 ml Methanol zu. Nach 12 Stunden Rühren bei Raumtemperatur wurde das Lösungsmittel entfernt, der Rückstand in Essigsäureethylester aufgenommen und die organische Phase viermal mit Wasser gewaschen. Nach Trocknen und Abdestillieren des Lösungsmittels erhielt man 3,60 g 2-Chlor-3-ethoxyiminomethyl-4-methylsulfonyl-benzoesäure (78 % Ausbeute).
   (Fp.: 155-160°C)
alternativ:
Stufe g') 2-Chlor-3-ethoxyiminomethyl-4-methylsulfonyl-benzoesäuremethylester (Verbindung 3.01)
   1,90 g (0,0195 mol) Ethoxyaminhydrochlorid und 1,35 g (0,0097 mol) fein gepulvertes Kaliumcarbonat wurden in 60 ml trockenem Methanol 1 Stunde bei Raumtemperatur gerührt und anschließend 4,90 g (0,0177 mol) 2-Chlor-3-formyl-4-methylsulfonyl-benzoesäuremethylester hinzugegeben. Nach 8 Stunden Rühren bei Raumtemperatur wurde das Lösungsmittel entfernt, der Rückstand in Essigsäureethylester aufgenommen, die organische Phase mit Wasser neutralgewaschen, getrocknet und am Vakuum eingeengt. Man erhielt 5,00 g 2-Chlor-3-ethoxyiminomethyl-4-methylsulfonyl-benzoesäuremethylester. (Ausbeute 88 %).
   (¹H-NMR (CDCl₃, δ in ppm): 1,34 (t, 3H); 3,29 (s, 3H); 3,98 (s, 3H); 4,26 (q, 2H); 7,91 (d, 1H); 8,10 (d, 1H); 8,38 (s, 1H).)
Stufe h') 2-Chlor-3-ethoxyiminomethyl-4-methylsulfonyl-benzoesäure
   Zu 7,29 g (0,055 mol) Lithiumiodid in 50 ml trockenem Pyridin wurde langsam eine Lösung von 4,37 g (0,0137 mol) 2-Chlor-3-ethoxyiminomethyl-4-methylsulfonyl-benzoesäuremethylester getropft. Nach 2 Stunden Rühren unter Rückfluß kühlte man das Reaktionsgemisch ab und entfernte das Lösungsmittel am Vakuum. Der Rückstand wurde in Wasser aufgenommen und mit verdünnter Salzsäure auf pH = 1-2 gestellt. Nach Extraktion der wäßrigen Phase mit Essigsäureethylester wurden die vereinigten organischen Phasen mit Wasser gewaschen, getrocknet und am Vakuum eingeengt. Man erhielt 3,70 g 2-Chlor-3-ethoxyiminomethyl-4-methylsulfonyl-benzoesäure. (Ausbeute 89 %).
   (Fp.: 155-160°C)

### 2-Chlor-3-chlorcarbonyl-4-methylsulfonyl-benzoesäuremethylester

Stufe a) 2-Chlor-3-hydroxycarbonyl-4-methylsulfonyl-benzoesäuremethylester
   Zu einer Lösung von 115,3 g (0,42 mol) 2-Chlor-3-formyl-4-methylsulfonyl-benzoesäuremethylester und 2000 ml Acetonitril wurden bei 5°C nacheinander 13,8 g (0,11 mol) Natriumhydrogenphosphatmonohydrat in 170 ml Wasser, 49,3 g (0,43 mol) 30 %ige Wasserstoffperoxid-lösung und 66,2 g (0,59 mol) 80 %ige wäßrige Natriumchloritlösung gegeben. Die Reaktionslösung wurde anschließend 1 Stunde bei 5°C und 12 Stunden bei Raumtemperatur gerührt. Dann wurde mit 10 %iger Salzsäure auf pH = 1 eingestellt und 1500 ml wäßrige 40 %ige Natriumhydrogensulfit-Lösung zugegeben. Nach 1 Stunde Rühren bei Raumtemperatur wurde die wäßrige Phase dreimal mit Essigsäureethylester extrahiert. Die vereinigten organischen Phasen wurden mit Natriumhydrogensulfit-Lösung gewaschen und getrocknet. Nach Abdestillation des Lösungsmittels erhielt man 102,0 g 2-Chlor-3-hydroxycarbonyl-4-methylsulfonyl-benzoesäuremethylester.
   (¹H-NMR (d⁶-DMSO, δ in ppm): 3,34 (s, 3H); 3,93 (s, 3H); 8,08 (s, 2H); 14,50 (s, br., 1H).)
Stufe b) 2-Chlor-3-chlorcarbonyl-4-methylsulfonyl-benzoesäuremethylester
   Zu einer Lösung von 6,0 g (0,021 mol) 2-Chlor-3-hydroxycarbonyl-4-methylsulfonyl-benzoesäuremethylester und 50 ml trockenem Toluol wurden 2 Tropfen Dimethylformamid und 11,9 g (0,1 mol) Thionylchlorid gegeben. Die Lösung wurde 4 Stunden unter Rückfluß erhitzt. Nach Entfernen des Lösungsmittels am Vakuum erhielt man 6,2 g 2-Chlor-3-chlorcarbonyl-4-methylsulfonyl-benzoesäuremethylester.
   (¹H-NMR (CDCl₃; δ in ppm): 3,21 (s, 3H); 4,02 (s, 3H); 8,02 (d, 1H); 8,07 (d, 1H).)

### 2,4-Dichlor-3-(1'-methoxyimino-1'-(methoxy)methyl)-benzoylchlorid (Verbindung 3.14)

Stufe a) 2,4-Dichlor-3-methyl-acetophenon
   Zu einer Lösung von 502,0 g (3,12 mol) 2,6-Dichlortoluol und 408,0 g (3,06 mol) Aluminiumtrichlorid wurden bei 100°C unter Rühren 235,0 g (3,0 mol) Acetylchlorid über einen Zeitraum von 2 Stunden zugetropft. Nach 2 Stunden Rühren bei 100-105°C kühlte man ab und goß das Reaktionsgemisch auf 3 1 Eis und 1 1 Wasser. Der dabei ausgefallene Feststoff wurde abgesaugt und mit 800 ml Wasser neutral gewaschen. Nach dem Trocknen bei 40°C erhielt man 500,0 g 2,4-Dichlor-3-methyl-acetophenon als Rohprodukt, das anschließend im Hochvakuum destilliert wurde.
   (Sdp.: 121-128°C (4 mbar))
Stufe b) 2,4-Dichlor-3-methyl-benzoesäure
   In eine Lösung von 520,0 g (13 mol) Natriumhydroxid in 2600 ml Wasser wurden bei 0-10°C zunächst 655.2 g (4,1 mol) Brom und anschließend 203,0 g (1,0 mol) 2,4-Dichlor-3-methyl-acetophenon in 1300 ml 1,4-Dioxan zugetropft. Nach 12 Stunden Rühren trennte man die organische Phase ab, versetzte die wäßrige Phase mit einer 30 %igen Lösung, dargestellt aus Natriumpyrosulfit und Wasser, und stellte mit Salzsäure einen pH-Wert von 1 ein. Der ausgefallene Niederschlag wurde abgesaugt, mit Wasser gewaschen und am Vakuum bei 60°C getrocknet. Man erhielt 197,0 g 2,4-Dichlor-3-methyl-benzoesäure.
   (Fp.: 173-175°C)
Stufe c) 2,4-Dichlor-3-methyl-benzoesäuremethylester
   Zu einer Lösung von 424,0 g (2 mol) 2,4-Dichlor-3-methylbenzoesäure und 1500 ml Methanol wurden 60 ml konz. Schwefelsäure getropft. Nach 5 Stunden Erhitzen unter Rückfluß wurde das Reaktionsgemisch abgekühlt, am Vakuum eingeengt und anschließend in 1000 ml Methylenchlorid aufgenommen. Die organische Phase wurde mit Wasser, anschließend mit 5 %iger Natriumhydrogencarbonat-Lösung und dann wiederum mit Wasser gewaschen, getrocknet und am Vakuum eingeengt. Man erhielt 401,0 g 2,4-Dichlor-3-methyl-benzoesäuremethylester.
   (Sdp: 103-107°C (1-1,5 mbar))
Stufe d) 3-Brommethyl-2,4-dichlor-benzoesäuremethylester
   Zu einer Lösung von 84,0 g (0,38 mol) 2,4-Dichlor-3-methyl-benzoesäuremethylester und 67,6 g (0,38 mol) N-Bromsuccinimid in 380 ml Tetrachlorkohlenstoff wurde 1,0 g Azobisisobutyronitril gegeben. Nach 3,5 Stunden Erhitzen unter Rückfluß wurde das Reaktionsgemisch abgekühlt und der gebildete Niederschlag abgesaugt. Das Filtrat wurde am Vakuum eingeengt und der resultierende Rückstand aus Methyl-tert.butylether ausgerührt. Man erhielt 108,0 g 3-Brommethyl-2,4-dichlor-benzoesäuremethylester.
   (Fp.: 51-54°C)
Stufe e) 2,4-Dichlor-3-formyl-benzoesäuremethylester
   Zu einer Lösung von 312,0 g (0,99 mol) 3-Brommethyl-2,4-dichlor-benzoesäuremethylester in 2 1 Acetonitril wurden unter Rückfluß 696,2 g (2,97 mol) wäßrige 50 %ige N-Methylmorpholin-N-oxid-Lösung getropft. Nach 48 Stunden Rühren bei Raumtemperatur wurde die Reaktionslösung in 6 1 Wasser eingerührt. Der ausgefallene Niederschlag wurde abgesaugt, mit wasser gewaschen und am Vakuum getrocknet. Man erhielt 141,3 g 2,4-Dichlor-3-formylbenzoesäuremethylester.
   (¹H-NMR (CDCl₃, δ in ppm): 3,98 (s, 3H); 7,47 (d, 1H); 7,84 (d, 1H); 10,48 (s, 1H).)
Stufe f) 2,4-Dichlor-3-hydroxycarbonyl-benzoesäuremethylester
   Zu einer Lösung von 40,0 g (0,172 mol) 2,4-Dichlor-3-formyl-benzoesäuremethylester und 500 ml Acetonitril wurden bei 5°C nacheinander 5,9 g (0,043 mol) Natriumdihydrogenphosphatmonohydrat in 70 ml Wasser, 20,5 g (0,181 mol) 30 %ige Wasserstoffperoxid-Lösung und 27,3 g (0,241 mol) 80 %ige Natriumchloritlösung gegeben. Die Reaktionslösung wurde 1 Stunde bei 5°C und 12 Stunden bei Raumtemperatur gerührt. Anschließend wurde mit 10 %iger Salzsäure ein pH-Wert von 1 eingestellt und 500 ml 40 %ige Natriumhydrogensulfit-Lösung zugegeben. Nach 1 Stunde Rühren bei Raumtemperatur wurde die wäßrige Phase dreimal mit Essigsäureethylester extrahiert, die vereinigten organischen Phasen mit 1,0 l 10 %iger Natriumhydrogensulfit-Lösung gewaschen und anschließend getrocknet. Nach Abdestillieren des Lösungsmittels erhielt man 40,0 g 2,4-Dichlor-3-hydroxycarbonyl-benzoesäuremethylester.
   (¹H-NMR (d⁶-DMSO, δ in ppm): 3,90 (s, 3H); 7,69 (d, 1H); 7,89 (d, 1H).)
Stufe g) 3-Chlorcarbonyl-2,4-dichlor-benzoesäuremethylester
   Zu einer Lösung von 5,00 g (0,02 mol) 2,4-Dichlor-3-hydroxycarbonyl-benzoesäuremethylester und 50 ml trockenem Toluol wurden 2 Tropfen Dimethylformamid und 11,90 g (0,1 mol) Thionylchlorid gegeben. Die Lösung wurde 4 Stunden unter Rückfluß erhitzt. Nach Abdestillieren des Lösungsmittels erhielt man 5,35 g 3-Chlorcarbonyl-2,4-dichlor-benzoesäuremethylester.
Stufe h) 2,4-Dichlor-3-methoxyaminocarbonyl-benzoesäuremethylester
   Zu einer Lösung von 5,35 g (0,02 mol) 3-Chlorcarbonyl-2,4-dichlor-benzoesäuremethylester und 100 ml Dichlormethan wurden 4,60 g (0,045 mol) Triethylamin und 3,75 g (0,045 mol) Methoxyaminhydrochlorid gegeben. Nach 12 Stunden Rühren bei Raumtemperatur wurde die Reaktionslösung mit verdünnter Phosphorsäure gewaschen, getrocknet und eingeengt. Der erhaltene Rückstand wurde in Diethylether ausgerührt. Man erhielt 4,80 g 2,4-Dichlor-3-methoxyaminocarbonyl-benzoesäuremethylester.
   (Fp.: 162-164°C)
Stufe i) 2,4-Dichlor-3-(1'-methoxyimino-1'-(methoxy)methyl)-benzoesäuremethylester (Verbindung 3.09)
   Ein Gemisch von 16,0 g (0,058 mol) 2,4-Dichlor-3-methoxyaminocarbonyl-benzoesäuremethylester und 10,1 g (0,073 mol) Kaliumcarbonat in 300 ml Dimethylformamid wurde 30 Minuten bei Raumtemperatur gerührt. Anschließend wurden 11,0 g (0,087 mol) Dimethylsulfat zugetropft, 12 Stunden bei Raumtemperatur gerührt und erneut 11,0 g Dimethylsulfat zugegeben. Nach 6 Stunden Erhitzen auf 60°C wurde das Reaktionsgemisch abgekühlt und in 2 1 Eiswasser eingerührt. Nun wurde die wäßrige Phase mit Essigsäureethylester extrahiert, die vereinigten organischen Phasen getrocknet und das Lösungsmittel am Vakuum abdestilliert. Nach Chromatographie des Rückstands an Kieselgel (Eluent: Toluol/Essigsäureethylester = 9/1) erhielt man 2,0 g 2,4-Dichlor-3-(1'-methoxyimino-1'-(methoxy)methyl)-benzoesäuremethylester.
   ⁽¹H-NMR (CDCl₃, δ in ppm): 3,43 (s, 3H); 3,58 (s, 3H); 3,92 (s, 3H); 7,35 (d, 1H); 7,82 (d, 1H).)
Stufe j) 2,4-Dichlor-3-(1'-methoxyimino-1'-(methoxy)methyl)benzoesäure (Verbindung 3.10)
   Eine Lösung von 2,20 g (0,008 mol) 2,4-Dichlor-3-(1'-methoxyimino-1'-(methoxy)methyl)benzoesäuremethylester und 3,00 g (0,075 mol) Natriumhydroxid in 50 ml Wasser wurde 2 Stunden bei 80°C gerührt. Nach Abkühlen wurde das Reaktionsgemisch in 200 ml Eiswasser eingerührt und mit konzentrierter Salzsäure auf pH = 1 gestellt. Die wäßrige Phase wurde mit Essigsäureethylester extrahiert, die vereinigten organischen Phasen getrocknet und am Vakuum eingeengt. Man erhielt 2,10 g 2,4-Dichlor-3-(1'-methoxyimino-1'-(methoxy)methyl)benzoesäure.
   ¹H-NMR (d⁶-DMSO, δ in ppm): 3,53 (s, 3H); 3,72 (s, 3H); 7,74 (d, 1H); 7,95 (d, 1H).)
Stufe k) 2,4-Dichlor-3-(1'-methoxyimino-1'-(methoxy)methyl)-benzoylchlorid (Verbindung 3.14)
   Eine Lösung von 2,10 g (0,0076 mol) 2,4-Dichlor-3-(1'-(methoxy)imino-1'-methoxymethyl)benzoesäure und 20,00 g Thionylchlorid in 50 ml trockenem Toluol wurde 2 Stunden bei 80°C gerührt. Nach dem Entfernen des Lösungsmittels am Vakuum erhielt man 2,25 g 2,4-Dichlor-3-(1'-methoxyimino-1'-(methoxy)methyl)benzoylchlorid

### 2,4-Dichlor-3-propoxyaminocarbonyl-benzoesäuremethylester

Zu einer Lösung von 4,50 g (0,04 mol) Propoxyaminhydrochlorid und 4.05 g (0,04 mol) Triethylamin in 200 ml Methylenchlorid wurden langsam bei 30°C 10,7 g (0,04 mol) 3-Chlorcarbonyl-2,4-dichlorbenzoesäuremethylester in 100 ml Methylenchlorid getropft. Nach 2 Stunden Rühren bei Raumtemperatur wurde das Reaktionsgemisch mit verdünnter Phosphorsäure gewaschen, getrocknet und eingeengt. Der erhaltene Rückstand wurde am Kieselgel chromatographiert (Eluent: Toluol/Essigsäureethylester = 9/1). Man erhielt 11,50 g 2,4-Dichlor-3-propoxyaminocarbonyl-benzoesäuremethylester.
(Fp.: 80-81°C)

### 3-(4-Chlorbenzyloxyaminocarbonyl)-2,4-dichlor-benzoesäuremethylester

Zu einer Lösung von 7,76 g (0,04 mol) 4-Chlorbenzyloxyaminhydrochlorid und 4,05 g (0,04 mol) Triethylamin in 200 ml Methylenchlorid wurden langsam bei ca. 30°C 10,70 g (0,04 mol) 3-Chlorcarbonyl-2,4-dichlor-benzoesäuremethylester in 50 ml Methylenchlorid getropft. Nach 12 Stunden Rühren bei Raumtemperatur wurde das Reaktionsgemisch mit verdünnter Phosphorsäure gewaschen, getrocknet und eingeengt. Nach Ausrühren des Rückstandes mit Diethylether erhielt man 19,00 g 3-(4-Chlorbenzyloxyaminocarbonyl)-2,4-dichlor-benzoesäuremethylester.
(Fp.: 120-121°C)

### 3-(1'-Methoxyiminoeth-1'-yl)-2-methyl-4-methylsulfonyl-benzoesäure (Verbindung 3.22)

Stufe a) 3-(1'-Methoxyiminoeth-1'-yl)-2-methyl-anilin
   50,0 g (0,335 mol) 3-Amino-2-methyl-acetophenon, 66,3 g (0,838 mol) Pyridin und 42,0 g (0,503 mol) O-Methylhydroxylamin-Hydrochlorid wurden in 400 ml Ethanol bei Raumtemperatur gerührt. Nach Entfernen des Lösungsmittels wurde der Rückstand in Methylenchlorid aufgenommen und mit Wasser gewaschen, getrocknet und eingeengt. Man erhielt 54,0 g (91 % d.Th.) 3-(1'-Methoxyiminoeth-1'-yl)-2-methyl-anilin.
Stufe b) 3-(1'-Methoxyiminoeth-1'-yl)-2-methyl-4-rhodano-anilin
   Zu 54,0 g (0,303 mol) 3-(1'-Methoxyiminoeth-1'-yl)-2-methyl-anilin, 49,3 g (0,479 mol) Natriumbromid und 77,5 g (0,956 mol) Natriumrhodanid in 300 ml Methanol wurden bei -20 bis -15°C 50,9 g (0,319 mol) Brom zugetropft. Nach 30 Minuten Rühren bei dieser Temperatur wurden die unlöslichen Bestandteile abgesaugt, das Filtrat mit Essigsäureethylester versetzt und mit wäßriger Natriumhydrogencarbonat-Lösung ein pH-Wert von 8 eingestellt. Die organische Phase wurde abgetrennt und die verbleibende wäßrige Phase mehrmals mit Essigsäureethylester extrahiert. Die vereinigten organischen Phasen wurden dann mit Wasser gewaschen, getrocknet und eingeengt. Man erhielt 67,3 g (95 % d.Th.) 3-(1'-Methoxyiminoeth-1'-yl)-2-methyl-4-rhodanoanilin.
Stufe c) 3-(1'-Methoxyiminoeth-1'-yl)-2-methyl-4-methylthioanilin
   Zu 40,4 g (0,315 mol) Natriumsulfid in 200 ml Wasser wurden bei 20 bis 30°C 67,3 g (0,286 mol) 3-(1'-Methoxyiminoeth-1'-yl)-2-methyl-4-rhodano-anilin in 600 ml Methanol getropft. Nach 3 Stunden Rühren bei Raumtemperatur wurden 45,1 g (0,318 mol) Methyliodid in 200 ml Methanol, ebenfalls bei 20 bis 30°C zugegeben. Anschließend wurde 12 Stunden bei Raumtemperatur gerührt, das Lösungsmittel entfernt, der Rückstand in Wasser aufgenommen und mehrmals mit Essigsäureethylester extrahiert. Die vereinigten organischen Phasen wurden dann mit Wasser gewaschen, getrocknet, eingeengt und der so erhaltene Rückstand mit n-Hexan/Methyl-tert.-butylether digeriert. Man erhielt 43,2 g (67 % d.Th.) 3-(1'-Methoxyiminoeth-1'-yl)-2-methyl-4-methylthio-anilin.
   (Fp.: 83-89°C)
Stufe d) 6-Brom-2-(1'-methoxyiminoeth-1'-yl)-3-methylthio-toluol
   Zu 3,00 g (13,4 mmol) 3-(1'-Methoxyiminoeth-1'-yl)-2-methyl-4-methylthio-anilin in 13,40 g Eisessig wurden bei Raumtemperatur 9,23 g 47 %ige Bromwasserstoffsäure zugetropft. Anschließend wurden 9,23 g Wasser zugegeben, 10 Minuten bei Raumtemperatur gerührt und bei -5 bis 0°C 0,92 g (13,4 mmol) Natriumnitrit in 1,9 ml Wasser zugegeben. Das resultierende Reaktionsgemisch wurde dann bei 0°C zu 1,92 g (13,4 mmol) Kupfer-(I)-bromid in 6 ml 47 %iger Bromwasserstoffsäure getropft. Nach 12 Stunden Rühren bei Raumtemperatur wurde auf Eiswasser gegossen und mit Methylenchlorid extrahiert. Die organische Phase wurde dann mit NatriumsulfitLösung und Wasser gewaschen, getrocknet und das Lösungsmittel entfernt. Man erhielt 2,50 g (65 % d.Th.) 6-Brom-2-(1'-methoxyiminoeth-1'-yl)-3-methylthiotoluol.
Stufe e) 6-Brom-2-(1'-methoxyiminoeth-1'-yl)-3-methylsulfonyltoluol
   Zu 2,5 g (8,71 mmol) 6-Brom-2-(1'-methoxyimino-1'-yl)-3-methylthio-toluol in 50 ml Methylenchlorid wurden innerhalb von 96 Stunden in Portionen insgesamt 7,0 g (34,80 mmol) m-Chlorperbenzoesäure gegeben. Nach Entfernen des Lösungsmittels wurde in organischem Lösungsmittel aufgenommen, eine Natriumcarbonatlösung, Natriumsulfitlösung und Wasser gewaschen, getrocknet und eingeengt. Der Rückstand wurde dann an Kieselgel (Eluent: Toluol/Essigsäureethylester) chromatographiert. Man erhielt 0,8 g (29 % d.Th.) 6-Brom-2-(1'-methoxyiminoeth-1'-yl)-3-methylsulfonyltoluol.
Stufe f) 3-(1'-Methoxyiminoeth-1'-yl)-2-methyl-4-methylsulfonylbenzoesäure
   0,77 g (2,41 mmol) 6-Brom-2-(1'-methoxyiminoeth-1'-yl)-3-methylsulfonyl-toluol, 0,03 g (0,1 mmol) Palladiumacetat, 0,14 g (0,49 mmol) Tricyclohexylphosphin, 0,10 g (2,4 mmol) Lithiumchlorid und 0,49 g (4,81 mmol) Triethylamin wurden in 37,5 ml Toluol und 17,5 ml Wasser suspendiert und bei 140°C, bei einem Druck von 20 bar 36 Stunden begast. Anschließend wurden nach Abkühlen die unlöslichen Bestandteile abgetrennt, die organische Phase mit Wasser (das mit 1 ml Triethylamin versetzt wurde) extrahiert, die resultierende wäßrige Phase mit Salzsäure auf pH = 1 gestellt und mit Methylenchlorid extrahiert. Diese organische Phase wurde getrocknet und eingeengt. Man erhielt 0,62 g (90 % d.Th.) 3-(1'-Methoxyiminoeth-1'-yl)-2-methyl-4-methylsulfonyl-benzoesäure.

In nachfolgender Tabelle 3 sind neben den voranstehend beschriebenen Verbindungen weitere Benzoesäurederivate der Formel IIIa aufgeführt, die in analoger Weise hergestellt wurden oder herstellbar sind.

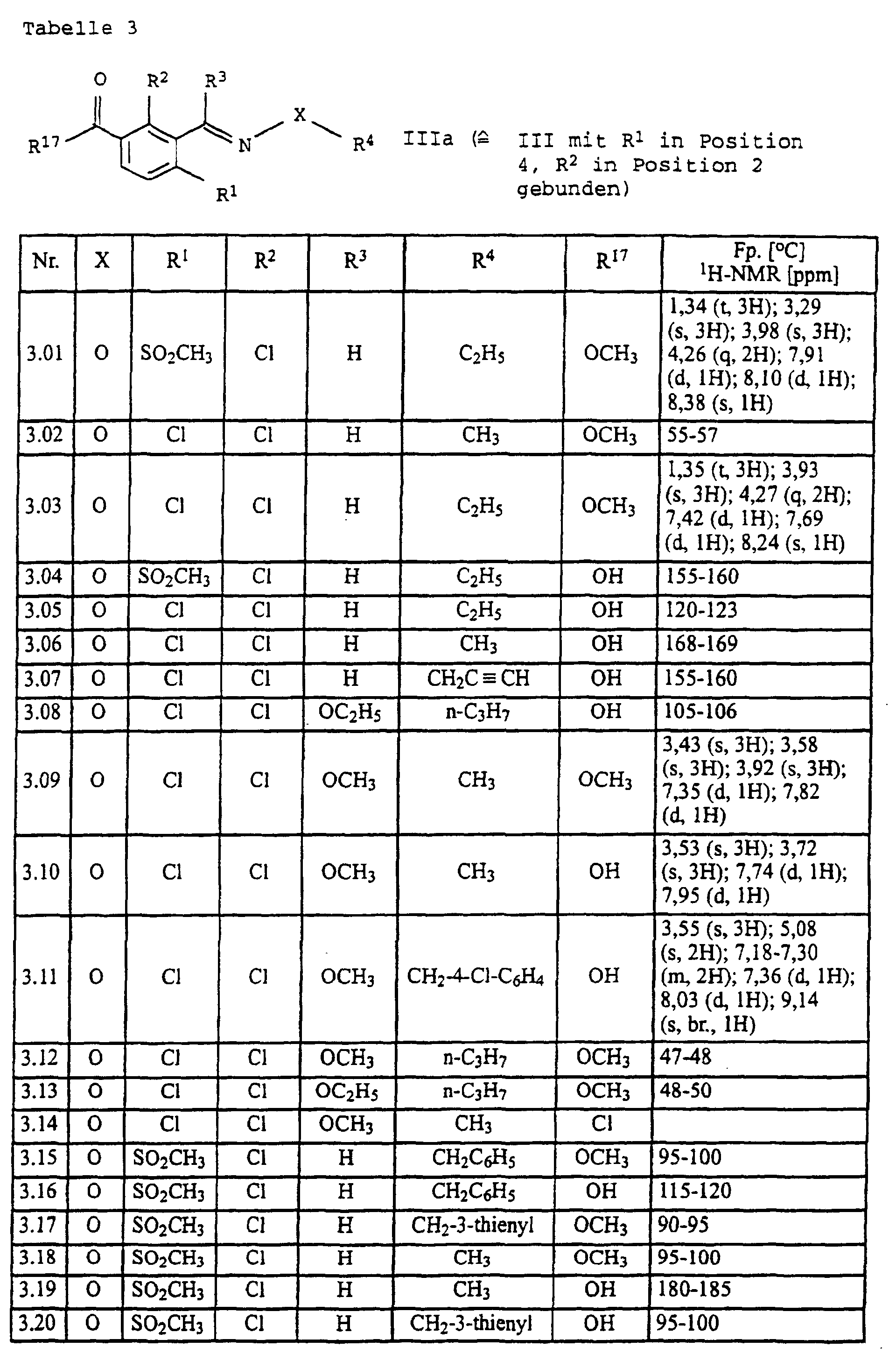

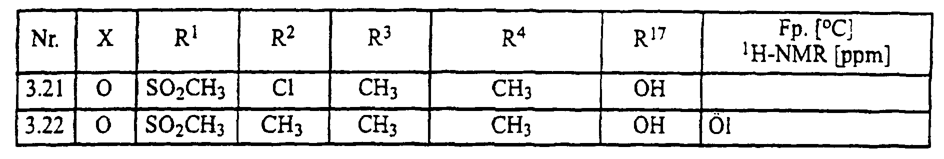

Die 2-Benzoyl-cyclohexan-1,3-dione der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmittel übliche Hilfsmittel.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
- I.: 20 Gewichtsteile der Verbindung Nr. 2.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
- II.: 20 Gewichtsteile der Verbindung Nr. 2.03 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III.: 20 Gewichtsteile des Wirkstoffs Nr. 2.05 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- IV.: 20 Gewichtsteile des Wirkstoffs Nr. 2.07 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
- V.: 3 Gewichtsteile des Wirkstoffs Nr. 2.10 werden mit 97 Gewichtsteilen feinteiligem Kaolin vermischt. Man erhält auf diese Weise ein Stäubemittel, das 3 Gew.-% des Wirkstoffs enthält.
- VI.: 20 Gewichtsteile des Wirkstoffs Nr. 2.14 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII.: 1 Gewichtsteil der Verbindung 2.06 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII.: 1 Gewichtsteil der Verbindung 2.09 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Die Applikation der Wirkstoffe der Formel I bzw. der herbiziden Mittel bzw. kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenon-oximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 2-Benzoyl-cyclohexan-1,3-dione der Formel I ließ sich durch Gewächshausversuche zeigen:
Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,125 bzw. 0,0625 kg/ha a. S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Englischer Name | Deutscher Name |
|---|---|---|
| Chenopodium album | lambsquarters (goosefoot) | Weißer Gänsefuß |
| Echinochloa crus galli | barnyardgrass | Hühnerhirse |
| Sinapis alba | white mustard | Weißer Senf |
| Zea mays | Indian corn | Mais |

Bei Aufwendungen von 0,125 bzw. 0,0625 kg/ha (a. S.) zeigte die Verbindung 2.07 (Tabelle 2) im Nachauflauf eine sehr gute Wirkung gegen oben genannte mono- und dikotyle Schadpflanzen und gute Verträglichkeit in Mais.

## Patentansprüche

1. 2-Benzoyl-cyclohexan-1,3-dione der Formel I 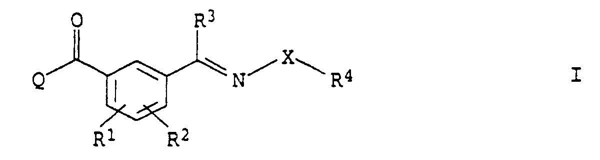 in der die Variablen folgende Bedeutung haben:
R¹, R² Wasserstoff, Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵, -OCOR⁶, -OSO₂R⁶, -SH, -S(O)ₙR⁷, -SO₂OR⁵, -SO₂NR⁵R⁸, - NR⁸SO₂R⁶ oder -NR⁸COR⁶;
R³ Wasserstoff, Cyano, C₁-C₆-Alkyl, C₁-C₆-Halogenalkyl, -OR⁷, -SR⁷ oder -NR⁷R¹⁰;
R⁴ Wasserstoff, C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
X Sauerstoff oder NR⁸;
n 0, 1 oder 2;
R⁵ Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁶ C₁-C₆-Alkyl oder C₁-C₆-Halogenalkyl;
R⁷ C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁸ Wasserstoff oder C₁-C₆-Alkyl;
R⁹ C₁-C₆-Alkyl, C₃-C₆-Alkenyl, C₃-C₆-Alkinyl, Phenyl oder Benzyl;
R¹⁰ C₁-C₆-Alkyl, C₁-C₆-Halogenalkyl, C₃-C₆-Alkenyl oder C₃-C₆-Alkinyl;
Q ein gegebenenfalls substituierter in 2-Stellung verknüpfter Cyclohexan-1,3-dionring;
sowie deren landwirtschaftlich brauchbaren Salze.

2. 2-Benzoyl-cyclohexan-1,3-dione der Formel I gemäß Anspruch 1, in der Q ein in 2-Stellung verknüpfter Cyclohexan-1,3-dionring der Formel II 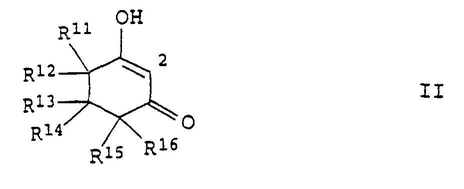 ist, wobei
R¹¹, R¹², R¹⁴ und R¹⁶ für Wasserstoff oder C₁-C₄-Alkyl stehen;
R¹³ für Wasserstoff, C₁-C₄-Alkyl oder C₃-C₄-Cycloalkyl steht, wobei die beiden letztgenannten Gruppen einen bis drei der folgenden Substituenten tragen können: Halogen, C₁-C₄-Alkylthio oder C₁-C₄-Alkoxy;
oder
für Tetrahydropyran-2-yl, Tetrahydropyran-3-yl, Tetrahydropyran-4-yl, Tetrahydrothiopyran-2-yl, Tetrahydrothiopyran-3-yl, Tetrahydrothiopyran-4-yl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die 6 letztgenannten Reste durch einen bis drei C₁-C₄-Alkylreste substituiert sein können;
R¹⁵ für Wasserstoff, C₁-C₄-Alkyl oder C₁-C₆-Alkoxycarbonyl steht;
oder
R¹³ und R¹⁶ gemeinsam eine π-Bindung oder einen drei- bis sechsgliedrigen carbocyclischen Ring bilden;
oder
die CR¹³R¹⁴-Einheit durch C=O ersetzt sein kann.

3. 2-Benzoyl-cyclohexan-1,3-dione der Formel I gemäß Anspruch 1 oder 2, in der
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷ bedeutet;
R² für Wasserstoff oder einen wie voranstehend unter R¹ genannten Rest steht.

4. 2-Benzoyl-cyclohexan-1,3-dione der Formel Ia 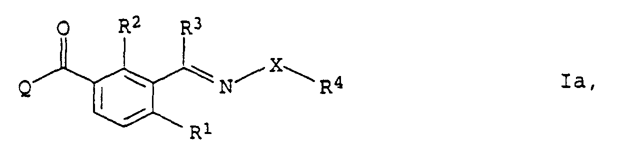 in der die Variablen R¹ bis R⁴, X und Q die unter den Ansprüchen 1 bis 3 genannte Bedeutung haben.

5. Verfahren zur Herstellung von 2-Benzoyl-cyclohexan-1,3-dionen der Formel I gemäß den Ansprüchen 1 bis 4, **dadurch gekennzeichnet, daß** man ein gegebenenfalls substituiertes Cyclohexan-1,3-dion Q mit einer aktivierten Carbonsäure IIIα oder mit einer Carbonsäure IIIβ, 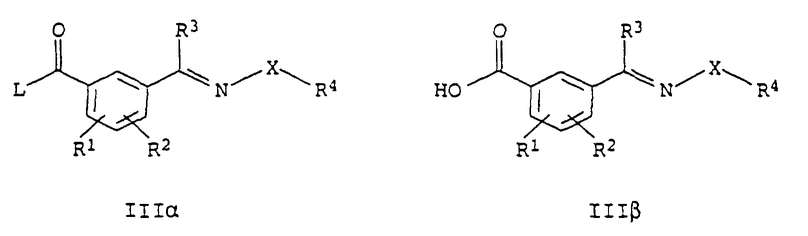 wobei die Variablen R¹ bis R⁴ und X die unter Anspruch 1 genannte Bedeutung haben und L für eine nucleophil verdrängbare Abgangsgruppe steht, acyliert und das Acylierungsprodukt gegebenenfalls in Gegenwart eines Katalysators zu den Verbindungen I umlagert.

6. Benzoesäurederivate der Formel IIIa, 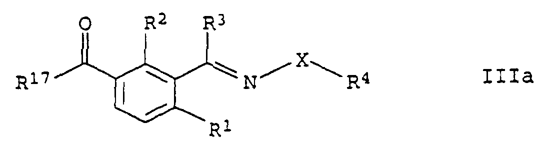 wobei
R¹ Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl oder -S(O)ₙR⁷;
R² Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁷;
R⁴ C₁-C₆-Alkyl, C₃-C₆-Cycloalkyl, C₃-C₆-Alkenyl, C₄-C₆-Cycloalkenyl, C₃-C₆-Alkinyl, -COR⁹, -CO₂R⁹, -COSR⁹ oder -CONR⁸R⁹, wobei die genannten Alkyl-, Cycloalkyl-, Alkenyl-, Cycloalkenyl- und Alkinylreste sowie R⁹ der Reste -COR⁹, -CO₂R⁹, -COSR⁹ und -CONR⁸R⁹ partiell oder vollständig halogeniert sein können und/oder eine bis drei der folgenden Gruppen tragen können:
Hydroxy, Mercapto, Amino, Cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-Alkyliminooxy, C₁-C₄-Alkoxyamino, C₁-C₄-Alkylcarbonyl, C₁-C₄-Alkoxy-C₂-C₆-alkoxycarbonyl, C₁-C₄-Alkylsulfonyl, Heterocyclyl, Heterocyclyloxy, Phenyl, Benzyl, Hetaryl, Phenoxy, Benzyloxy und Hetaryloxy, wobei die acht letztgenannten Reste ihrerseits substituiert sein können;
R¹⁷ für Hydroxy oder einen abhydrolysierbaren Rest steht
und die Variablen R³, R⁵ und R⁷ bis R¹⁰, X und n die unter Anspruch 1 genannte Bedeutung haben.

7. Benzoesäurederivate der Formel IIIa nach Anspruch 6,
wobei
R¹⁷ Halogen, Hydroxy oder C₁-C₆-Alkoxy bedeutet.

8. Mittel, enthaltend eine herbizid wirksame Menge mindestens einer Verbindung der Formel I oder eines landwirtschaftlich brauchbaren Salzes von I, gemäß den Ansprüchen 1 bis 4, und für die Formulierung von Pflanzenschutzmitteln übliche Hilfsmittel.

9. Verfahren zur Herstellung von herbizid wirksamen Mitteln gemäß Anspruch 8, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 4 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens einer Verbindung der Formel I oder eines landwirtschaftlich brauchbaren Salzes von I gemäß den Ansprüchen 1 bis 4 auf Pflanzen, deren Lebensraum und/ oder auf Samen einwirken läßt.

11. Verwendung der Verbindungen der Formel I und deren landwirtschaftlich brauchbaren Salze gemäß den Ansprüchen 1 bis 4 als Herbizide.

## Claims

1. 2-Benzoylcyclohexane-1,3-diones of the formula I 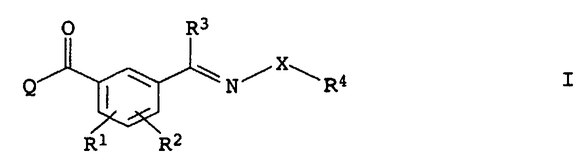 where:
R¹ and R² are each hydrogen, nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵, -OCOR⁶, -OSO₂R⁶, -SH, -S(O)ₙR⁷, -SO₂OR⁵, -SO₂NR⁵R⁸, -NR⁸SO₂R⁶ or -NR⁸COR⁶;
R³ is hydrogen, cyano, C₁-C₆-alkyl, C₁-C₆-haloalkyl, -OR⁷, -SR⁷ or -NR⁷R¹⁰;
R⁴ is hydrogen, C₁-C₆-alkyl, C₃-C₆-cycloalkyl, C₃-C₆-alkenyl, C₄-C₆-cycloalkenyl, C₃-C₆-alkynyl, -COR⁹, -CO₂R⁹, -COSR⁹ or -CONR⁸R⁹, where the alkyl, cycloalkyl, alkenyl, cycloalkenyl and alkynyl radicals mentioned and R⁹ of the radicals -COR⁹, -CO₂R⁹, -COSR⁹ and -CONR⁸R⁹ may be partially or fully halogenated and/or carry one to three of the following groups:
hydroxyl, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
X is oxygen or NR⁸;
n is 0, 1 or 2;
R⁵ is hydrogen, C₁-C₆-alkyl, C₁-C₆-haloalkyl, C₁-C₆-alkoxy-C₂-C₆-alkyl, C₃-C₆-alkenyl or C₃-C₆-alkynyl;
R⁶ is C₁-C₆-alkyl or C₁-C₆-haloalkyl;
R⁷ is C₁-C₆-alkyl, C₁-C₆-haloalkyl, C₁-C₆-alkoxy-C₂-C₆-alkyl, C₃-C₆-alkenyl or C₃-C₆-alkynyl;
R⁸ is hydrogen or C₁-C₆-alkyl;
R⁹ is C₁-C₆-alkyl, C₃-C₆-alkenyl, C₃-C₆-alkynyl, phenyl or benzyl;
R¹⁰ is C₁-C₆-alkyl, C₁-C₆-haloalkyl, C₃-C₆-alkenyl or C₃-C₆-alkynyl;
Q is a cyclohexane-1,3-dione ring attached in position 2 with or without substitution;
and agriculturally useful salts thereof.

2. 2-Benzoylcyclohexane-1,3-diones of the formula I as claimed in claim 1 in which Q is a cyclohexane-1,3-dione ring of the formula II 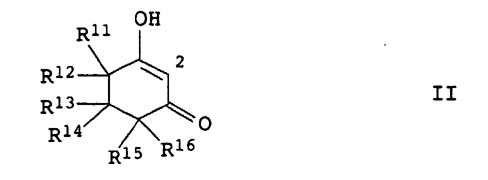 attached in position 2 where
R¹¹, R¹², R¹⁴ and R¹⁶ are each hydrogen or C₁-C₄-alkyl;
R¹³ is hydrogen, C₁-C₄-alkyl or C₃-C₄-cycloalkyl, where the last two groups may carry one to three of the following substituents:
halogen, C₁-C₄-alkylthio or C₁-C₄-alkoxy;
or
is tetrahydropyran-2-yl, tetrahydropyran-3-yl, tetrahydropyran-4-yl, tetrahydrothiopyran-2-yl, tetrahydrothiopyran-3-yl, tetrahydrothiopyran-4-yl, 1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, where the last 6 radicals mentioned may be substituted by one to three C₁-C₄-alkyl radicals;
R¹⁵ is hydrogen, C₁-C₄-alkyl or C₁-C₆-alkoxycarbonyl;
or
R¹³ and R¹⁶ together form a π-bond or a three- to six-membered carbocyclic ring;
or
the CR¹³R¹⁴ unit is replaced by C=O.

3. 2-Benzoylcyclohexane-1,3-diones of the formula I as claimed in claim 1 or 2, where
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵ or -S(O)ₙR⁷;
R² is hydrogen or one of the radicals mentioned above under R¹.

4. 2-Benzoylcyclohexane-1,3-diones of the formula Ia 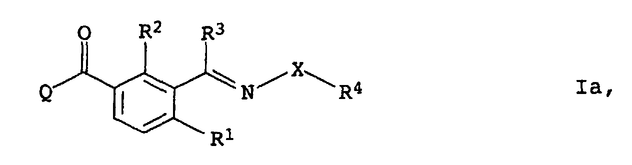 where the variables R¹ to R⁴, X and Q are each as defined under claims 1 to 3.

5. A process for preparing 2-benzoylcyclohexane-1,3-diones of the formula I as claimed in any of claims 1 to 4, which comprises acylating an unsubstituted or substituted cyclohexane-1,3-dione Q with an activated carboxylic acid IIIα or with a carboxylic acid IIIβ, 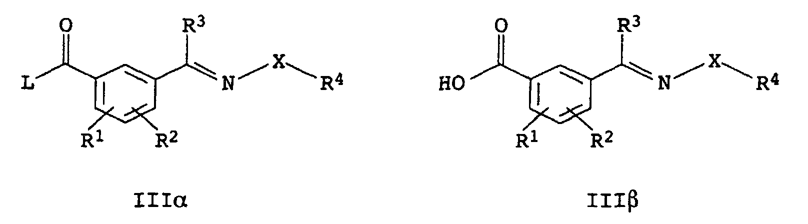 where the variables R¹ to R⁴ and X are each as defined under claim 1 and L is a nucleophilically displaceable leaving group, and rearranging the acylation product in the presence or absence of a catalyst to the compounds I.

6. Benzoic acid derivatives of the formula IIIa, 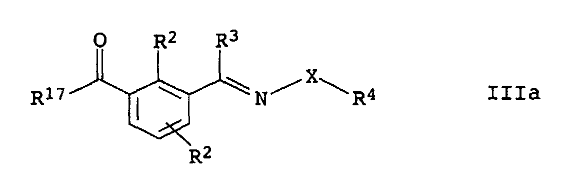 where
R¹ is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl or -S(O)ₙR⁷;
R² is nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵ or -S(O)ₙR⁷;
R⁴ is C₁-C₆-alkyl, C₃-C₆-cycloalkyl, C₃-C₆-alkenyl, C₄-C₆-cycloalkenyl, C₃-C₆-alkynyl, -COR⁹, -CO₂R⁹, -COSR⁹ or -CONR⁸R⁹, where the alkyl, cycloalkyl, alkenyl, cycloalkenyl and alkynyl radicals mentioned and R⁹ of the radicals -COR⁹, -CO₂R⁹, -COSR⁹ and -CONR⁸R⁹ may be partially or fully halogenated and/or carry one to three of the following groups:
hydroxyl, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, C₁-C₄-alkyliminooxy, C₁-C₄-alkoxyamino, C₁-C₄-alkylcarbonyl, C₁-C₄-alkoxy-C₂-C₆-alkoxycarbonyl, C₁-C₄-alkylsulfonyl, heterocyclyl, heterocyclyloxy, phenyl, benzyl, hetaryl, phenoxy, benzyloxy and hetaryloxy, where the last eight radicals mentioned may in turn be substituted;
where R¹⁷ is hydroxyl or a radical that can be removed by hydrolysis and the variables R³, R⁵ and R⁷ to R¹⁰, X and n are each as defined under claim 1.

7. Benzoic acid derivatives of the formula IIIa as claimed in claim 6,
where
R¹⁷ is halogen, hydroxyl or C₁-C₆-alkoxy.

8. A composition comprising a herbicidally active amount of at least one compound of the formula I or an agriculturally useful salt of I as claimed in any of claims 1 to 4, and auxiliaries conventionally used in the formulation of crop protection agents.

9. A process for preparing herbicidally active compositions as claimed in claim 8, which comprises mixing a herbicidally active amount of at least one compound of the formula I or an agriculturally useful salt of I as claimed in any of claims 1 to 4 and auxiliaries conventionally used in the formulation of crop protection agents.

10. A method for controlling undesirable vegetation, which comprises allowing a herbicidally active amount of at least one compound of the formula I or an agriculturally useful salt of I as claimed in any of claims 1 to 4 to act on plants, their habitat and/or on seeds.

11. Use of the compounds of the formula I and of agriculturally useful salts thereof as claimed in any of claims 1 to 4 as herbicides.

## Revendications

1. 2-benzoyl-cyclohexane-1,3-diones de formule I 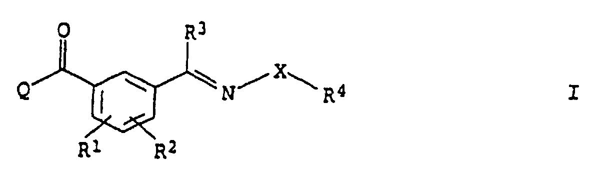 dans laquelle les symboles ont les significations suivantes :
R¹, R² : l'hydrogène, un groupe nitro, des halogènes, des groupes cyano, thiocyano, alkyle en C1-C6, halogénoalkyle en C1-C6, (alcoxy en C1-C6)alkyle en C1-C6, alcényle en C2-C6, alcynyle en C2-C6, -OR⁵, -OCOR⁶, -OSO₂R⁶, -SH, -S(O)ₙR⁷, -SO₂OR⁵, -SO₂NR⁵R⁸, -NR⁸SO₂R⁶ ou -NR⁸COR⁶ ;
R³ : l'hydrogène, un groupe cyano, alkyle en C1-C6, halogénoalkyle en C1-C6, -OR⁷, -SR⁷ ou -NR⁷R¹⁰ ;
R⁴ : l'hydrogène, un groupe alkyle en C1-C6, cycloalkyle en C3-C6, alcényle en C3-C6, cycloalcényle en C4-C6, alcynyle en C3-C6, -COR9, -CO₂R⁹, -COSR⁹ ou -CONR⁸R⁹, les groupes alkyle, cycloalkyle, alcényle, cycloalcényle et alcynyle en question, de même que les parties R⁹ des groupes -COR⁹, -CO₂R⁹, -COSR⁹ et -CONR⁸R⁹, pouvant être halogénés en totalité ou en partie et/ou pouvant porter de un à trois des substituants suivants : hydroxy, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, (alkyle en C1-C4)iminooxy, (alcoxy en C1-C4)amino, (alkyle en C1-C4)carbonyle, (alcoxy en C1-C4)-(alcoxy en C2-C6)carbonyle, alkylsulfonyle en C1-C4, hétérocyclyle, hétérocyclyloxy, phényle, benzyle, hétéroaryle, phénoxy, benzyloxy et hétéroaryloxy, les huit groupes mentionnés en dernier pouvant eux-mêmes être substitués ;
X : l'oxygène ou NR⁸ ;
n = 0,-1 ou 2 ;
R⁵ = l'hydrogène, un groupe alkyle en C1-C6, halogénoalkyle en C1-C6, (alcoxy en C1-C6)alkyle en C2-C6, alcényle en C3-C6 ou alcynyle en C3-C6 ;
R⁶ : un groupe alkyle en C1-C6 ou halogénoalkyle en C1-C6 ;
R⁷ : un groupe alkyle en C1-C6, halogénoalkyle en C1-C6, (alcoxy en C1-C6)alkyle en C2-C6, alcényle en C3-C6 ou alcynyle en C3-C6 ;
R⁸ : l'hydrogène ou un groupe alkyle en C1-C6 ;
R⁹ : un groupe alkyle en C1-C6, alcényle en C3-C6, alcynyle en C3-C6, phényle ou benzyle ;
R¹⁰ : un groupe alkyle en C1-C6, halogénoalkyle en C1-C6, alcényle en C3-C6 ou alcynyle en C3-C6 ;
Q : un noyau cyclohexane-1,3-dione éventuellement substitué, relié en position 2 ;
et leurs sels aptes aux applications agricoles.

2. 2-benzoyl-cyclohexane-1,3-diones de formule I de la revendication 1 dans laquelle Q représente un noyau cyclohexane-1,3-dione relié en position 2, qui répond à la formule II 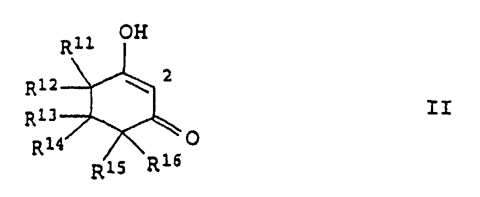 dans laquelle
R¹¹, R¹², R¹⁴ et R¹⁶ représentent l'hydrogène ou des groupes alkyle en C1-C4 ;
R¹³ représente l'hydrogène, un groupe alkyle en C1-C4 ou cycloalkyle en C3-C4, les deux groupes mentionnés en dernier pouvant porter un à trois des substituants suivants : halogéno, alkylthio en C1-C4 ou alcoxy en C1-C4;
ou bien
un groupe tétrahydropyran-2-yle, tétrahydropyran-3-yle, tétrahydropyran-4-yle, tétrahydrothiopyran-2-yle, tétrahydrothiopyran-3-yle, tétrahydrothiopyran-4-yle, 1,3-dioxolan-2-yle, 1,3-dioxan-2-yle, 1,3-oxathiolan-2-yle, 1,3-oxanthian-2-yle, 1,3-dithiolan-2-yle ou 1,3-dithian-2-yle, les six groupes mentionnés en dernier pouvant porter un à trois substituants alkyle en C1-C4 ;
R¹⁵ représente l'hydrogène, un groupe alkyle en C1-C4 ou (alcoxy en C1-C6)carbonyle ;
ou bien
R¹³ et R¹⁶ forment ensemble une liaison ou un noyau carboxylique de trois à six chaînons ;
ou bien
le motif CR¹³R¹⁴ peut être remplacé par C=O.

3. 2-benzoyl-cyclohexane-1,3-diones de formule I de la revendication 1 ou 2 dans laquelle
R¹ représente un groupe nitro, un halogène, un groupe cyano, thiocyano, alkyle en C1-C6, halogénoalkyle en C1-C6, (alcoxy en C1-C6)alkyle en C1-C6, alcényle en C2-C6, alcynyle en C2-C6, -OR⁵ ou -S(O)ₙR⁷ ;
R² représente l'hydrogène ou l'un des substituants mentionnés ci-dessus en référence à R¹.

4. 2-benzoyl-cyclohexane-1,3-diones de formule Ia 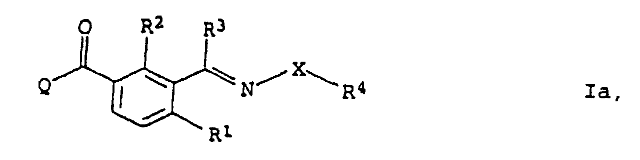 dans laquelle les symboles R¹ à R⁴, X et Q ont les significations indiquées dans les revendications 1 à 3.

5. Procédé de préparation des 2-benzoyl-cyclohexane-1,3-diones de formule I selon les revendications 1 à 4, **caractérisé par le fait que** l'on acyle une cyclohexane-1,3-dione éventuellement substituée Q par un acide carboxylique activé IIIα ou un acide carboxylique IIIβ, 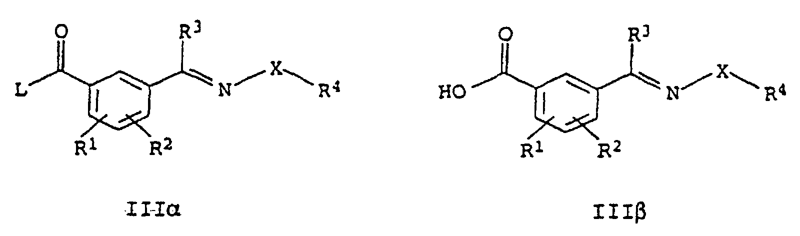 pour lesquels les symboles R¹ à R⁴ et X ont les significations indiquées dans la revendication 1 et L représente un substituant nucléophile éliminable, et on soumet le produit d'acylation à transposition en les composés I éventuellement en présence d'un catalyseur.

6. Dérivés de l'acide benzoïque répondant à la formule IIIa 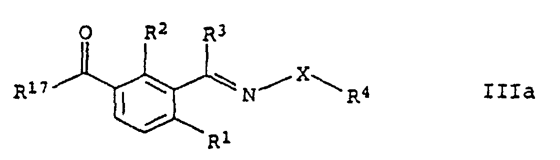 dans laquelle
R¹ représente un groupe nitro, un halogène, un groupe cyano, thiocyano, alkyle en C1-C6, halogénoalkyle en C1-C6, (alcoxy en C1-C6)alkyle en C1-C6, alcényle en C2-C6, alcynyle en C2-C6 ou -S(O)ₙR⁷ ;
R² représente un groupe nitro, un halogène, un groupe cyano, thiocyano, alkyle en C1-C6, halogénoalkyle en C1-C6, (alcoxy en C1-C6)alkyle en C1-C6, alcényle en C2-C6, alcynyle en C2-C6, -OR⁵ ou-S(O)ₙR⁷ ;
R⁴ représente un groupe alkyle en C1-C6, cycloalkyle en C3-C6, alcényle en C3-C6, cycloalcényle en C4-C6, alcynyle en C3-C6, -COR⁹, -CO₂R⁹, -COSR⁹ ou -CONR⁸R⁹, les groupes alkyle, cycloalkyle, alcényle, cycloalcényle et alcynyle en question, de même que les parties R⁹ des groupes -COR⁹, -CO₂R⁹, -COSR⁹ et -CONR⁸R⁹, pouvant être halogénés en totalité ou en partie et/ou pouvant porter un à trois des substituants suivants :
hydroxy, mercapto, amino, cyano, R¹⁰, -OR¹⁰, -SR¹⁰, -NR⁸R¹⁰, =NOR¹⁰, -OCOR¹⁰, -SCOR¹⁰, -NR⁸COR¹⁰, -CO₂R¹⁰, -COSR¹⁰, -CONR⁸R¹⁰, (alkyle en C1-C4)iminooxy, (alcoxy en C1-C4)amino, (alkyle en C1-C4)carbonyle, (alcoxy en C1-C4)-(alcoxy en C2-C6)carbonyle, alkylsulfonyle en C1-C4, hétérocyclyle, hétérocyclyloxy, phényle, benzyle, hétéroaryle, phénoxy, benzyloxy et hétéroaryloxy, les huit groupes mentionnés en dernier pouvant eux-mêmes être substitués ;
R¹⁷ représente un groupe hydroxy ou un substituant éliminable par hydrolyse, et les symboles R³, R⁵ et R⁷ à R¹⁰, X et n ont les significations indiquées dans la revendication 1.

7. Dérivés de l'acide benzoïque de formule IIIa selon la revendication 6, dans laquelle R¹⁷ représente un halogène, un groupe hydroxy ou alcoxy en C1-C6.

8. Produit contenant une quantité herbicide efficace d'au moins un composé de formule I ou de l'un de ses sels aptes aux applications agricoles selon les revendications 1 à 4, et des produits auxiliaires usuels pour la formulation des produits phytosanitaires.

9. Procédé pour la préparation de produits à activité herbicide selon la revendication 8, **caractérisé par le fait que** l'on mélange une quantité herbicide efficace d'au moins un composé de formule I ou de l'un de ses sels aptes aux applications agricoles selon les revendications 1 à 4 et des produits auxiliaires usuels pour la formulation des produits phytosanitaires.

10. Procédé pour combattre les croissances de végétaux indésirables, **caractérisé par le fait que** l'on fait agir une quantité herbicide efficace d'au moins un composé de formule I ou de l'un de ses sels aptes aux applications agricoles selon les revendications 1 à 4, sur les végétaux, leur habitat et/ou les semences.

11. Utilisation des composés de formule I et leurs sels aptes aux applications agricoles selon les revendications 1 à 4 en tant qu'herbicides.
